(19) **Europäisches Patentamt European Patent Office Office européen des brevets**

(11) **EP 4 737 570 A1**

(12) **EUROPEAN PATENT APPLICATION**
published in accordance with Art. 153(4) EPC

(43) Date of publication:
06.05.2026  Bulletin 2026/19

(21) Application number: 24832518.5

(22) Date of filing: 28.06.2024

(51) International Patent Classification (IPC):
*C12N 15/113* (2010.01)

(52) Cooperative Patent Classification (CPC):
**C12N 15/113; A61P 35/00; C12N 15/1135;**
C12N 2310/14; C12N 2310/321; C12N 2310/322;
C12N 2310/3521; C12N 2310/3533

(86) International application number:
**PCT/KR2024/009133**

(87) International publication number:
**WO 2025/005742 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 28.06.2023  KR 20230083349
28.06.2023  KR 20230083355
28.06.2023  KR 20230083360
27.06.2024  KR 20240084878

(71) Applicant: Exollence Co., Ltd.
Seoul 07532 (KR)

(72) Inventors:
• **CHUNG, Jihwa**
Seoul 07532 (KR)
• **KIM, Hyo Kyeong**
Seoul 07532 (KR)
• **CHOI, Yujeong**
Seoul 07532 (KR)

(74) Representative: **Dehns**
**10 Old Bailey**
**London EC4M 7NG (GB)**

Remarks:
The complete document including Reference
Table(s) and the Sequence Listing(s) can be
downloaded from the EPO website

(54) **PHARMACEUTICAL COMPOSITION FOR PREVENTING OR TREATING CANCER COMPRISING SIRNA AS ACTIVE INGREDIENT**

(57)    The present invention relates to a pharmaceutical composition for preventing or treating cancer, the composition comprising siRNA as an active ingredient. The pharmaceutical composition was found to significantly inhibit the cell proliferation effect of colorectal cancer or pancreatic cancer, thus having an excellent anticancer effect, and reduce the expression level of KRAS mRNA. In addition, the composition maintains the body weight of an individual even while reducing the size and weight of a tumor, and thus can be effectively used as an excellent anticancer therapeutic agent.

EP 4 737 570 A1

**FIG. 5A**

# EP 4 737 570 A1

**Description**

[Technical Field]

The present invention relates to a pharmaceutical composition for preventing or treating cancer comprising siRNA as an active ingredient.

This application claims priority to and the benefit of Korean Patent Application Nos. 10-2023-0083349, 10-2023-0083355, and 10-2023-0083360, each filed on June 28, 2023, the disclosure of which is incorporated herein by reference in its entirety.

Additionally, this application claims priority to and the benefit of Korean Patent Application No. 10-2024-0084878, filed on June 27, 2024, the disclosure of which is incorporated herein by reference in its entirety.

[Background Art]

Cancer is a genetic disease that forms tumors through abnormal division, and when it worsens, it becomes a malignant tumor, metastasizes to surrounding tissue and threatens life. The main cause of cancer is the abnormal overexpression of genes related to cell growth and division, or mutations in genes that control them.

There are various types of cancer depending on an organ in which cancer occurs, and according to statistical data from the Ministry of Health and Welfare, pancreatic cancer ranked eighth in all cancer cases occurring in 2017 and is known to have a poor prognosis because it is difficult to diagnose early and easily metastasizes to other organs. In addition, the 5-year survival rate for pancreatic cancer is approximately 12%, which is significantly lower than 76.5%, 75%, and 93% for stomach cancer, colon cancer, and breast cancer, respectively. Anticancer agents such as gemcitabine and radiation therapy, which are used today, can cause various side effects in the vascular system, nervous system, blood system and skin, so new treatments that minimize these side effects and have better therapeutic effects are needed.

Colorectal cancer is a malignant tumor (adenocarcinoma) that occurs in the colon or rectum, and is cancer that mostly occurs in the mucosa. Depending on where cancer occurs, when occurring in the colon, it is called colon cancer, and when occurring in the rectum, it is called rectal cancer. These types of cancer are collectively called colorectal cancer. According to data from the Central Cancer Registry published in 2017, 214,701 cancer cases occurred in Korea in 2015. Among them, colorectal cancer was the second most common, with 26,790 cases in both men and women, accounting for 12.5% of all cancer cases. It was more prevalent in men, with a male-to-female ratio of 1.5:1. Particularly, colorectal cancer is known to be prevalent, with 15,911 cases in men, ranking third among cancers in men, and 10,879 cases in women, also ranking third among cancers in women.

Meanwhile, gene therapy is a technology that uses genes to treat or prevent diseases and is actively being developed to treat intractable or incurable diseases such as cancer. Small interfering RNA (siRNA), one of the gene therapy technologies for cancer treatment, is a 21 to 23-bp long RNA duplex that suppresses gene expression by forming an RISC complex and cleaving mRNA. When the expression of genes essential for cancer development is suppressed using siRNA, cancer can be treated through the inhibition of cancer cell growth or death of cancer cells.

Despite the above methods, few effective siRNA therapeutics for treating cancer have been reported to date.

[Disclosure]

[Technical Problem]

An object of the present invention is to provide a pharmaceutical composition for preventing or treating cancer, including one or more selected from the group consisting of siRNAs having any one nucleotide sequence selected from the group consisting of SEQ ID NOs: 1 to 29 as an active ingredient.

Another object of the present invention is to provide a kit for preventing or treating cancer, comprising a composition including one or more selected from the group consisting of siRNAs having any one nucleotide sequence selected from the group consisting of SEQ ID NOs: 1 to 29 as an active ingredient, and an instruction manual.

However, technical problems to be solved in the present invention are not limited to the above-described problems, and other problems which are not described herein will be fully understood by those of ordinary skill in the art from the following descriptions.

[Technical Solution]

The present invention provides a pharmaceutical composition for preventing or treating cancer, comprising one or more selected from the group consisting of siRNAs having any one nucleotide sequence selected from the group consisting of SEQ ID NOs: 1 to 29 as an active ingredient.

In one embodiment of the present invention, the group consisting of siRNAs below may have a sense strand that is 19 to 25 nt long and an anti-sense strand that is 21 to 27 nt long, but is not limited thereto:

1) siRNA having any one nucleotide sequence selected from the group consisting of SEQ ID NOs: 1 to 5;
2) siRNA having a nucleotide sequence represented by SEQ ID NO: 7;
3) siRNA having any one nucleotide sequence selected from the group consisting of SEQ ID NOs: 14, 15, and 17; and
4) siRNA having any one nucleotide sequence selected from the group consisting of SEQ ID NOs: 20, 23, and 26 to 28.

In one embodiment of the present invention, the group consisting of siRNA having a nucleotide sequence represented by SEQ ID NO: 12, siRNA having a nucleotide sequence represented by SEQ ID NO: 13, siRNA having a nucleotide sequence represented by SEQ ID NO: 18, siRNA having a nucleotide sequence represented by SEQ ID NO: 21, siRNA having a nucleotide sequence represented by SEQ ID NO: 24, and siRNA having a nucleotide sequence represented by SEQ ID NO: 29 may have the following characteristics, but is not limited thereto:

a) having a sense strand that is 19 to 25 nt long and an anti-sense strand that is 21 to 27 nt long;
b) having no tt overhang in a sense strand;
c) having a 2'-OMe-chemically modified nucleotide at the 7th position from the 5' end of a sense strand, and a nucleotide in an anti-sense strand binding to this position, which is not chemically modified with 2'-OMe;
d) having a nucleotide at the 9th position from the 5' end of the sense strand, which is not chemically modified with 2'-OMe; and
e) having 2'-OMe-chemically modified nucleotides at the 18th and 20th positions from the 5' end of the anti-sense strand.

In one embodiment of the present invention, the group consisting of siRNA comprising the nucleotide sequence represented by SEQ ID NO: 6, siRNA comprising the nucleotide sequence represented by SEQ ID NO: 8, siRNA comprising the nucleotide sequence represented by SEQ ID NO: 9, siRNA comprising the nucleotide sequence represented by SEQ ID NO: 10, siRNA comprising the nucleotide sequence represented by SEQ ID NO: 11, siRNA comprising the nucleotide sequence represented by SEQ ID NO: 16, siRNA comprising the nucleotide sequence represented by SEQ ID NO: 19, siRNA comprising the nucleotide sequence represented by SEQ ID NO: 22, and siRNA comprising the nucleotide sequence represented by SEQ ID NO: 25 may have the following characteristics, but is not limited thereto:

a) having a sense strand that is 19 to 25 nt long and an anti-sense strand that is 21 to 27 nt long;
b) having a 2'-OMe-chemically modified nucleotide at the 7th position from the 5' end of a sense strand, and a nucleotide in an anti-sense strand binding to this position, which is not chemically modified with 2'-OMe;
c) having a nucleotide at the 9th position from the 5' end of the sense strand, which is not chemically modified with 2'-OMe; and
d) having 2'-OMe-chemically modified nucleotides at the 16th, 18th and 20th positions from the 5' end of the anti-sense strand.

In one embodiment of the present invention, the cancer may be one or more selected from the group consisting of pancreatic cancer, colorectal cancer, squamous cell carcinoma, lung cancer, adenocarcinoma of the lung, peritoneal cancer, skin cancer, skin or intraocular melanoma, rectal cancer, anal cancer, esophageal cancer, small intestinal cancer, endocrine cancer, parathyroid cancer, adrenal cancer, soft tissue sarcoma, urethral cancer, blood cancer, liver cancer, gastric cancer, glioblastoma, cervical cancer, ovarian cancer, bladder cancer, liver tumors, breast cancer, colon cancer, endometrial cancer, uterine cancer, salivary gland cancer, kidney cancer, prostate cancer, vulvar cancer, thyroid cancer, head and neck cancer, and brain cancer, but is not limited thereto.

In one embodiment of the present invention, the composition may inhibit the proliferation of cancer cells, but is not limited thereto.

In one embodiment of the present invention, the composition may reduce tumor size and weight, but is not limited thereto.

In one embodiment of the present invention, the composition may inhibit KRAS mRNA expression, but is not limited thereto.

The present invention provides a kit for preventing or treating cancer, including a composition comprising one or more selected from the group consisting of siRNAs having any one nucleotide sequence selected from the group consisting of SEQ ID NOs: 1 to 29 as an active ingredient, and an instruction manual.

Additionally. The present invention provides a method of treating cancer, or preventing or treating pancreatic cancer or colorectal cancer, comprising:

administering a pharmaceutically effective amount of a composition comprising one or more selected from the group consisting of siRNAs having any one nucleotide sequence selected from the group consisting of SEQ ID NOs: 1 to 29 as an active ingredient to a subject in need thereof.

Additionally. The present invention provides a use of a composition comprising one or more selected from the group consisting of siRNAs having any one nucleotide sequence selected from the group consisting of SEQ ID NOs: 1 to 29 as an active ingredient for preventing or treating cancer, or preventing or treating pancreatic cancer or colorectal cancer,.

Additionally. The present invention provides a use of a composition comprising one or more selected from the group consisting of siRNAs having any one nucleotide sequence selected from the group consisting of SEQ ID NOs: 1 to 29 as an active ingredient for preparing a formulation for preventing or treating cancer, or preventing or treating pancreatic cancer or colorectal cancer.

Additionally, the present invention provides a method of preventing or treating cancer, or preventing or treating pancreatic cancer or colorectal cancer, comprising:

administering a pharmaceutically effective amount of a composition comprising one or more siRNAs selected from each of the following groups as an active ingredient to a subject in need thereof,
wherein the siRNAs are selected from:

i) a group consisting of siRNA comprising any one nucleotide sequence selected from the group consisting of SEQ ID NOs: 1 to 5; siRNA comprising a nucleotide sequence represented by SEQ ID NO: 7; siRNA comprising any one nucleotide sequence selected from the group consisting of SEQ ID NOs: 14, 15, and 17; and siRNA comprising any one nucleotide sequence selected from the group consisting of SEQ ID NOs: 20, 23, and 26 to 28;
ii) a group consisting of siRNA comprising a nucleotide sequence represented by SEQ ID NO: 12; siRNA comprising a nucleotide sequence represented by SEQ ID NO: 13; siRNA comprising a nucleotide sequence represented by SEQ ID NO: 18; siRNA comprising a nucleotide sequence represented by SEQ ID NO: 21; siRNA comprising a nucleotide sequence represented by SEQ ID NO: 24; and siRNA comprising a nucleotide sequence represented by SEQ ID NO: 29; or
iii) a group consisting of siRNA comprising a nucleotide sequence represented by SEQ ID NO: 6; siRNA comprising a nucleotide sequence represented by SEQ ID NO: 8; siRNA comprising a nucleotide sequence represented by SEQ ID NO: 9; siRNA comprising a nucleotide sequence represented by SEQ ID NO: 10; siRNA comprising a nucleotide sequence represented by SEQ ID NO: 11; siRNA comprising a nucleotide sequence represented by SEQ ID NO: 16; siRNA comprising a nucleotide sequence represented by SEQ ID NO: 19; siRNA comprising a nucleotide sequence represented by SEQ ID NO: 22; and siRNA comprising a nucleotide sequence represented by SEQ ID NO: 25.

Additionally, the present invention provides a use for preparing a formulation for preventing or treating cancer, or for preventing or treating pancreatic cancer or colorectal cancer, comprising at least one siRNA selected from each of the above groups as an active ingredient.

Additionally, the present invention provides a use for preparing a formulation for preventing or treating cancer, or for preventing or treating pancreatic cancer or colorectal cancer, comprising one or more siRNAs selected from each of the above groups as an active ingredient.

Additionally, the present invention provides a use for preparing a formulation for preventing or treating cancer, or for preventing or treating pancreatic cancer or colorectal cancer, comprising one or more siRNAs selected from each of the above groups as an active ingredient.

[Advantageous Effects]

According to the pharmaceutical composition for preventing or treating cancer comprising siRNA as an active ingredient, it was found to significantly inhibit the cell proliferation effect of colorectal cancer or pancreatic cancer, thereby exhibiting excellent anticancer effects and reducing the expression level of KRAS mRNA. Additionally, since it reduces the size and weight of tumors while maintaining the body weight of the subject, it may be usefully employed as an excellent anticancer therapeutic agent.

[Description of Drawings]

FIG. 1A shows a single graph ranking the results obtained by analyzing and summarizing the cell death rates when 105 types of siRNA candidate materials were treated on three pancreatic cancer cell lines and three colorectal cancer cell lines.

FIG. 1B confirms the cell death effects when the 105 types of siRNA candidate materials were treated on three pancreatic cancer cell lines.

FIG. 1C confirms the cell death effects when the 105 types of siRNA candidate materials were treated on three colorectal cancer cell lines.

FIG. 1D shows the sequences and modifications of the 29 siRNA candidate materials of the present invention.

FIG. 1E shows the sequences (ss strand and as strand) of the 29 siRNAs identified as lead substances among the 105 siRNA candidate materials.

FIGS. 2A to 2G confirm the cell death effects when the 29 lead siRNAs were treated on three pancreatic cancer cell lines.

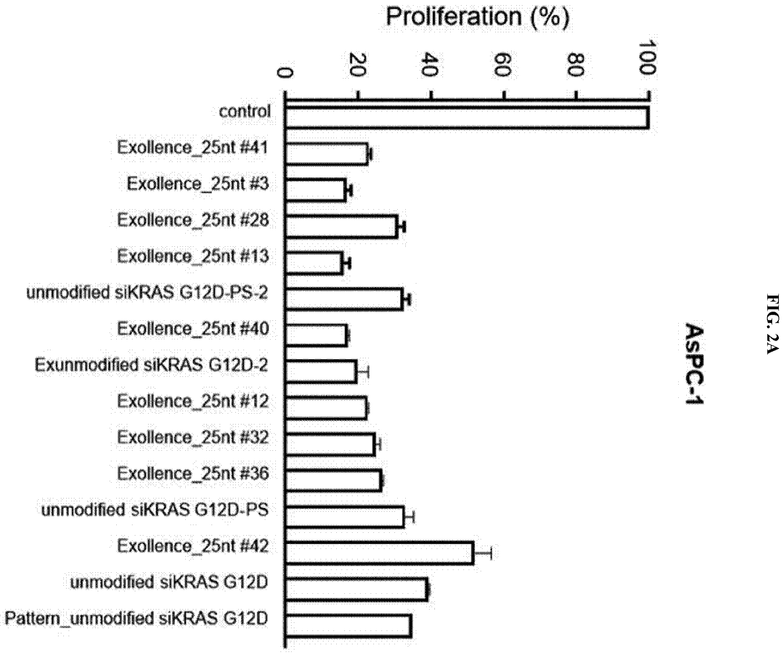

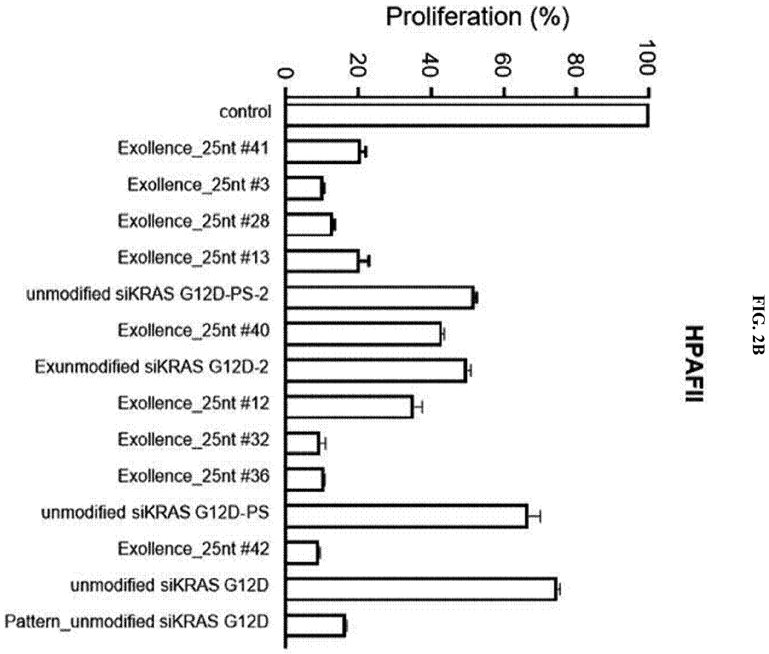

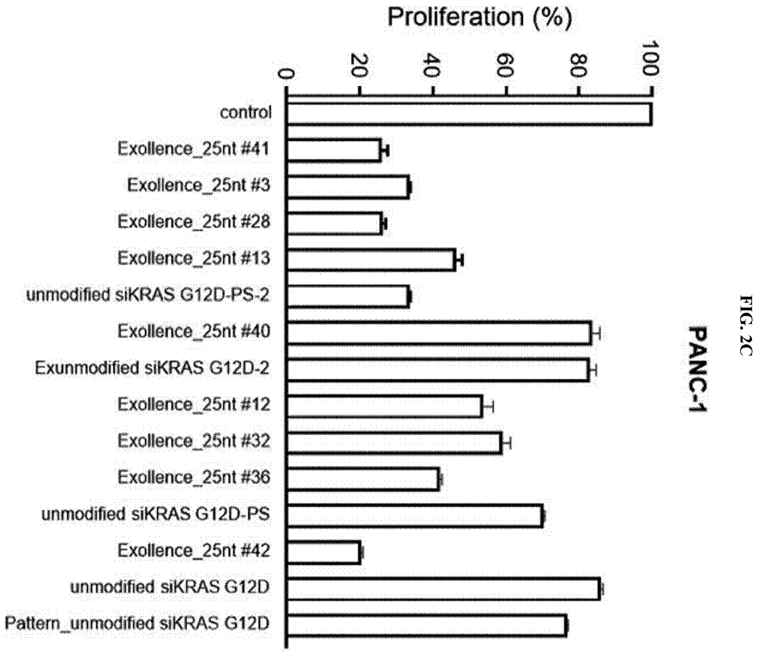

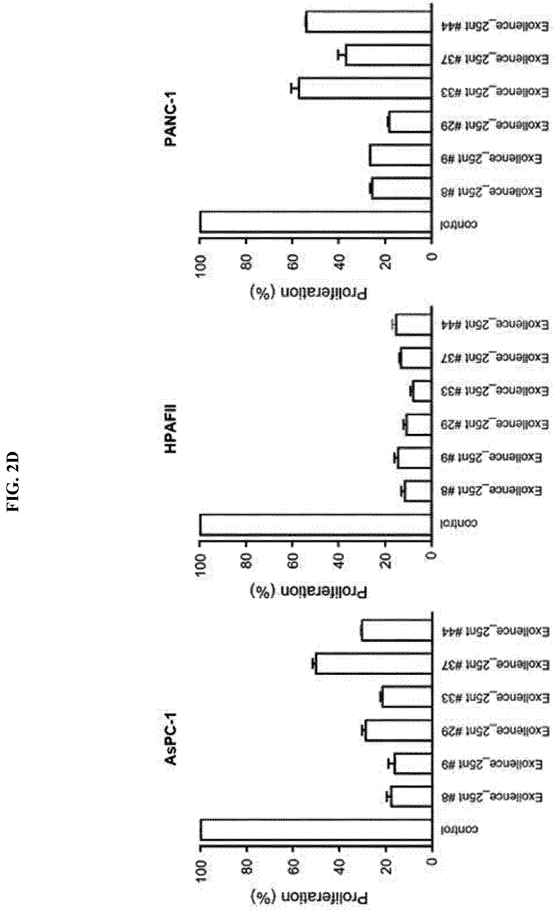

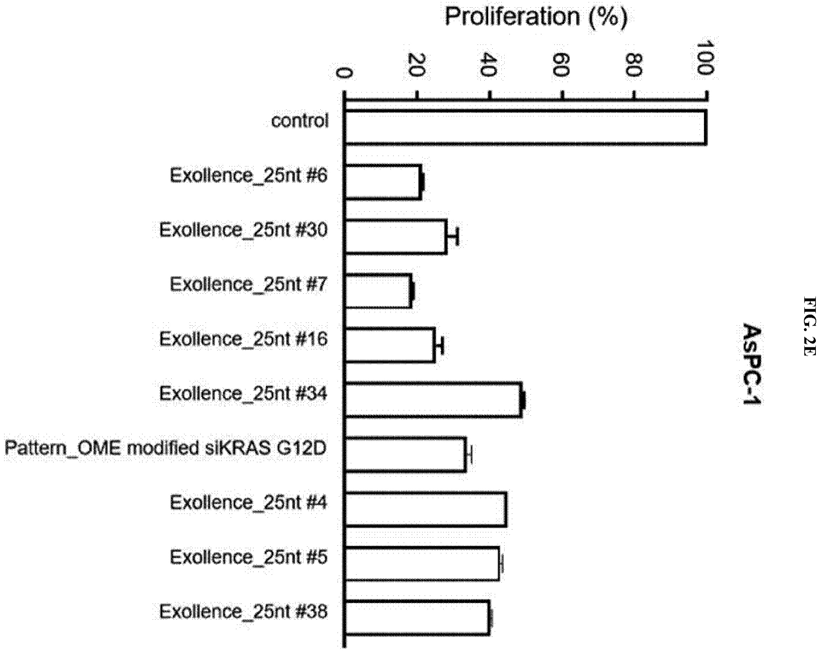

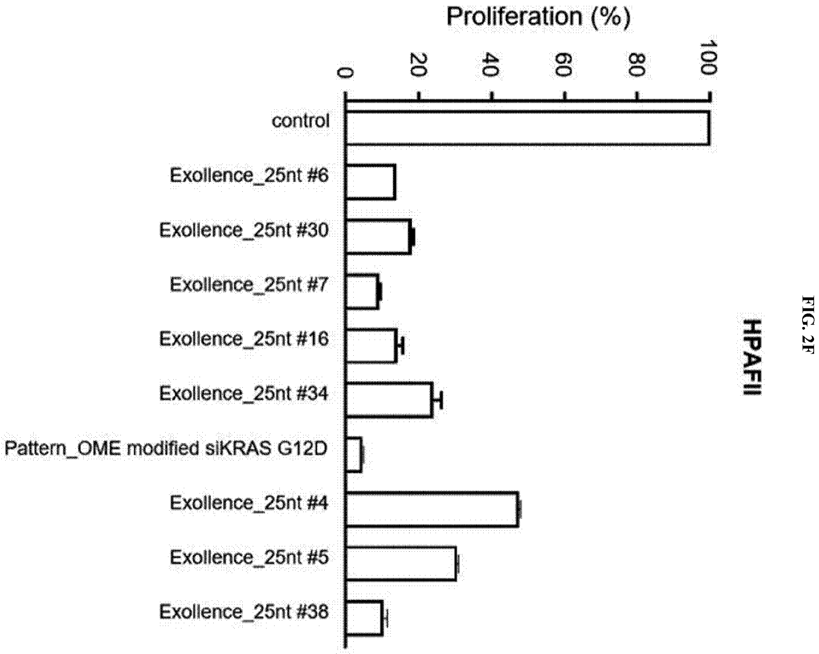

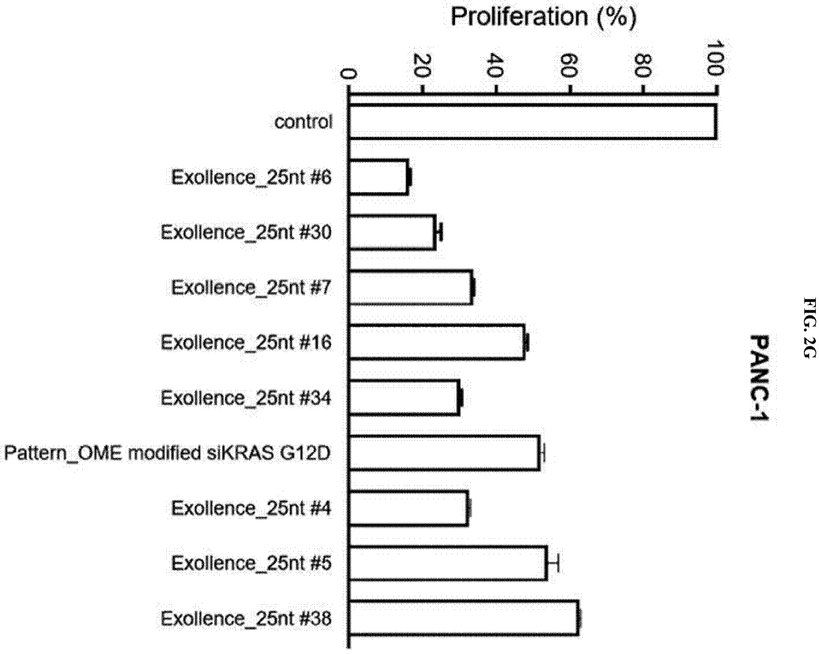

FIGS. 2A to 2C respectively show the experimental results for the lead substances of Group 1, FIG. 2D shows those of Group 2, and FIGS. 2E to 2G show those of Group 3.

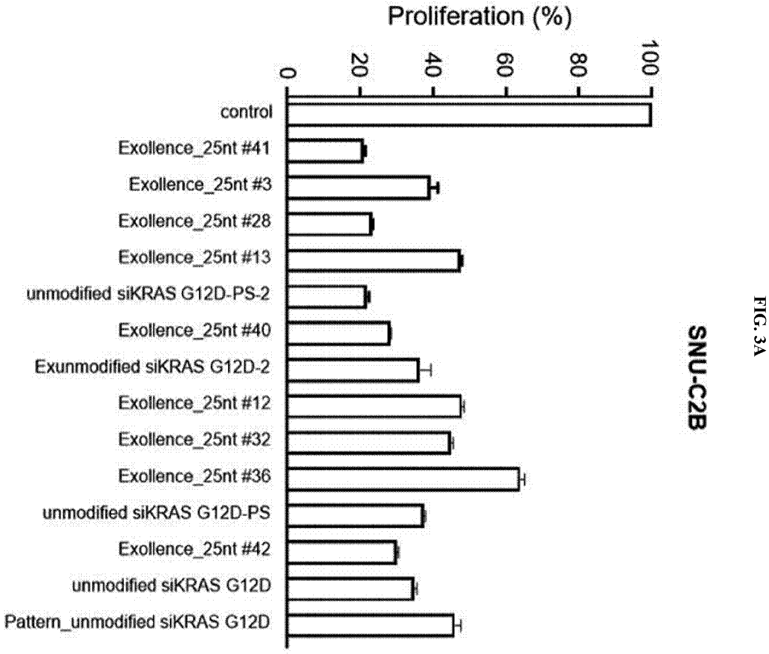

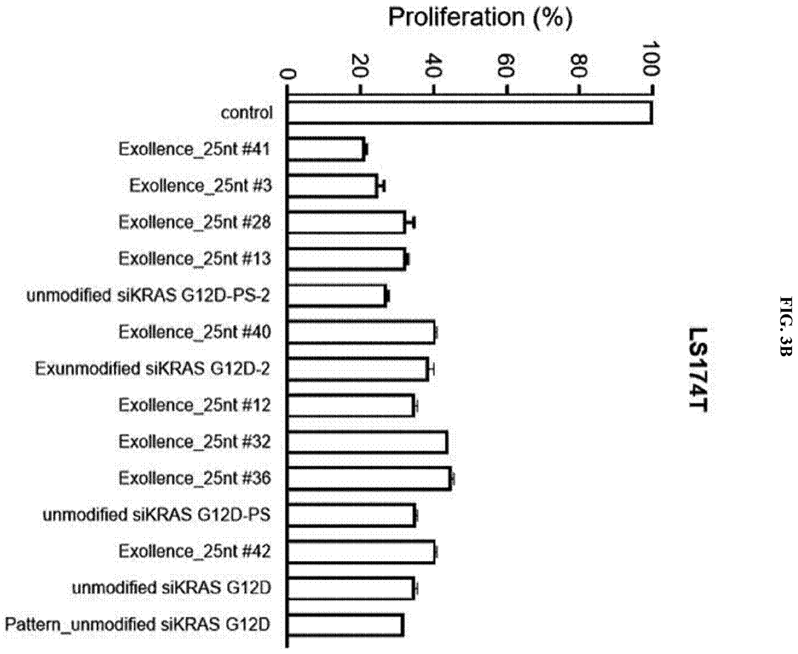

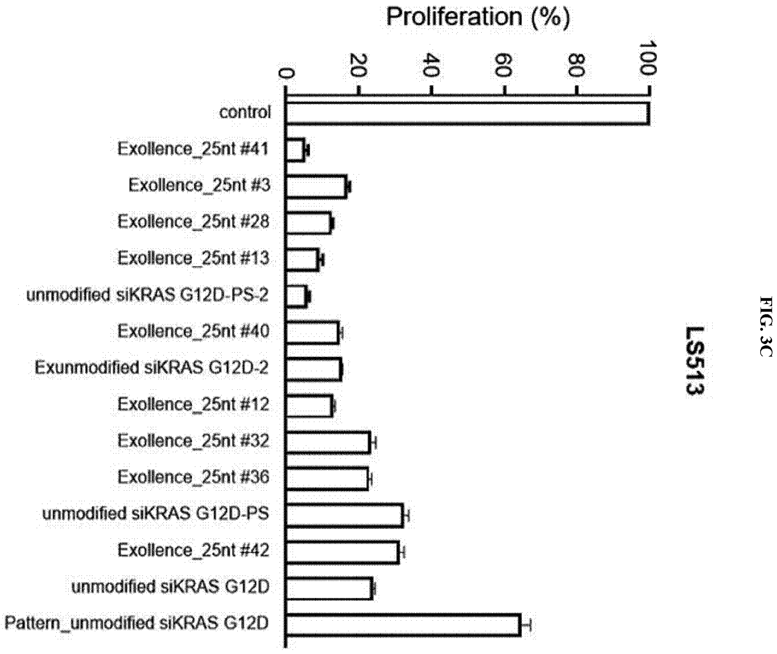

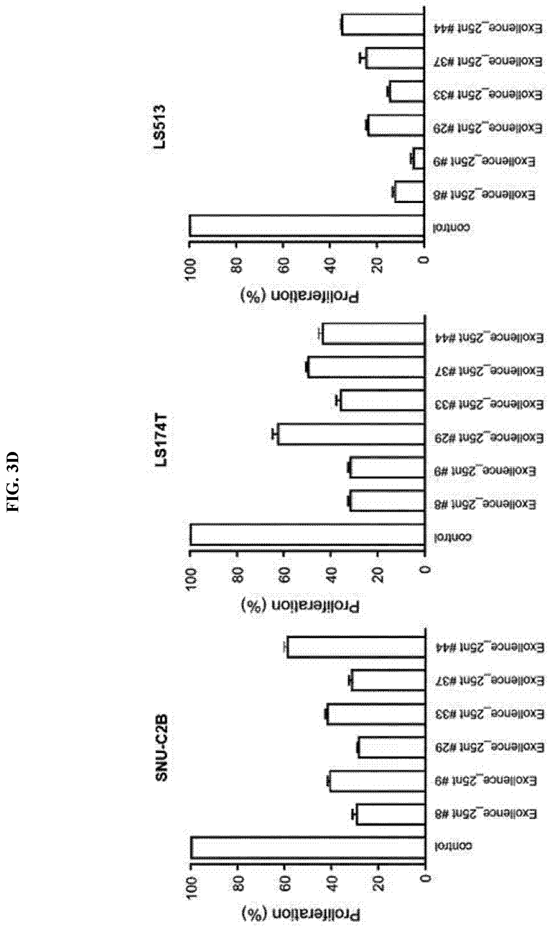

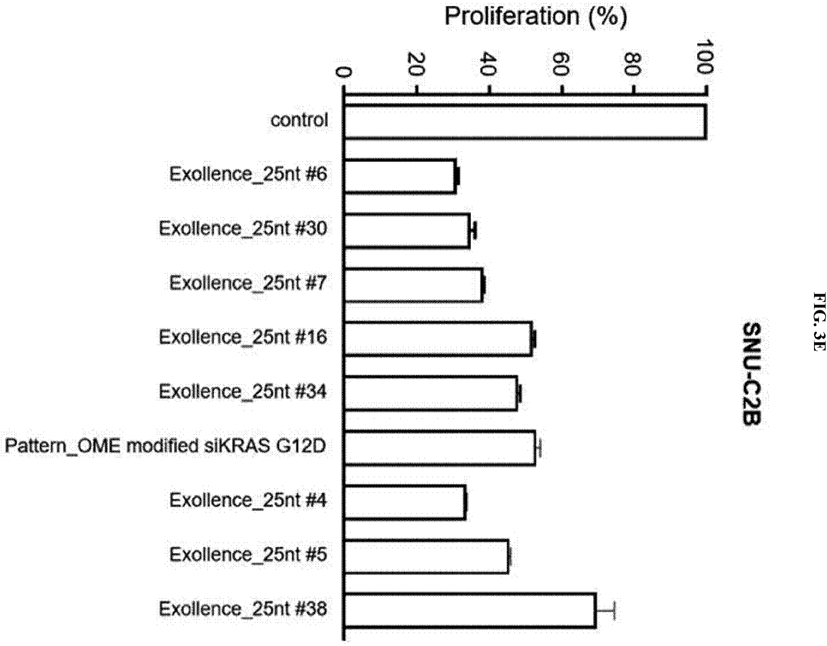

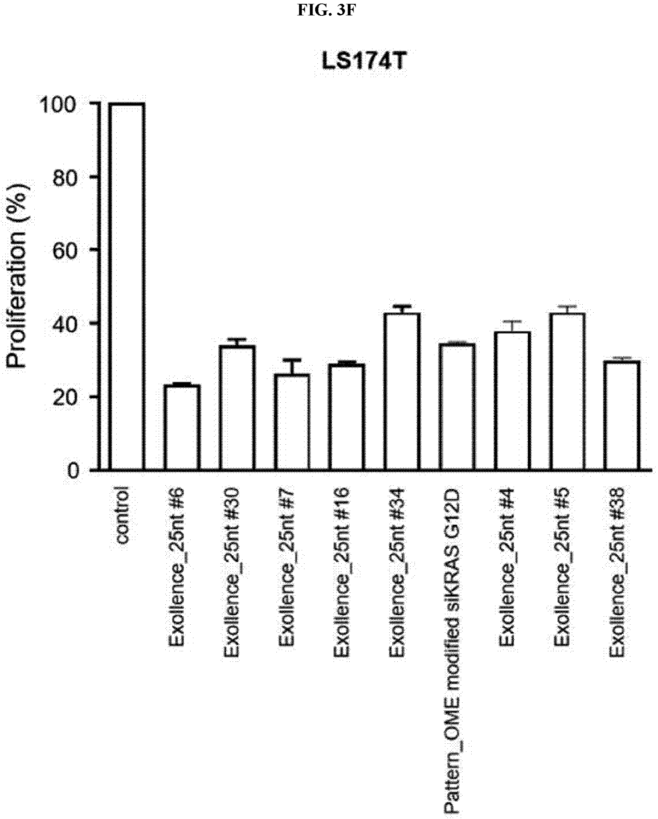

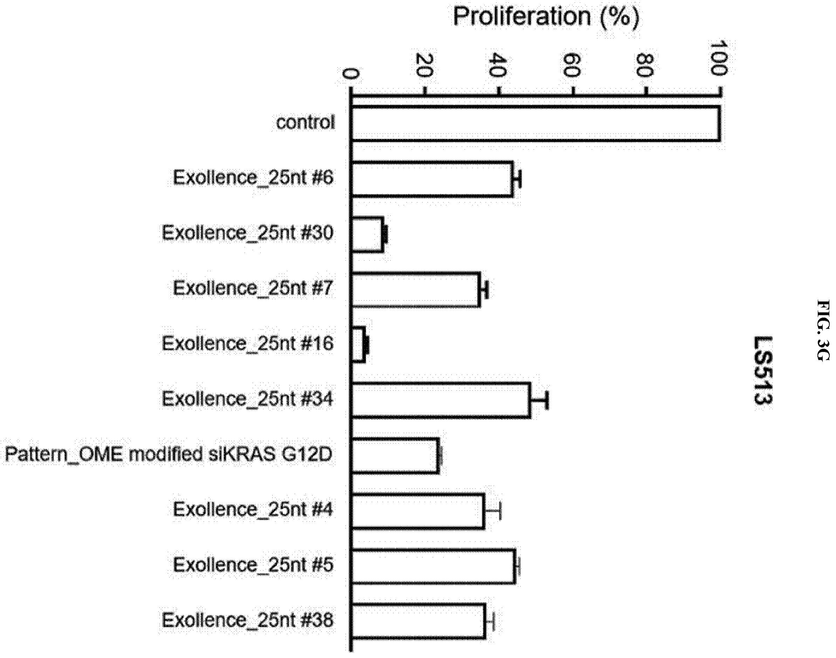

FIGS. 3A to 3G confirm the cell death effects when the 29 lead siRNAs were treated on three colorectal cancer cell lines. FIGS. 3A to 3C respectively show the experimental results for the lead substances of Group 1, FIG. 3D shows those of Group 2, and FIGS. 3E to 3G show those of Group 3.

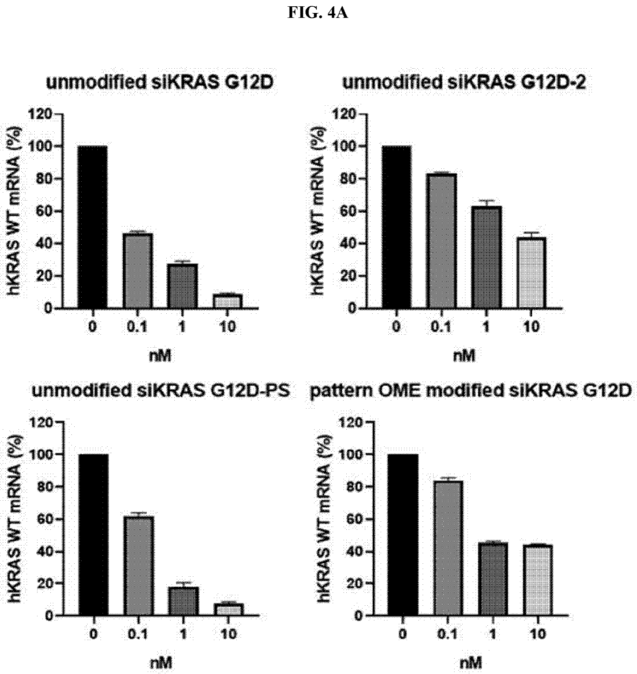

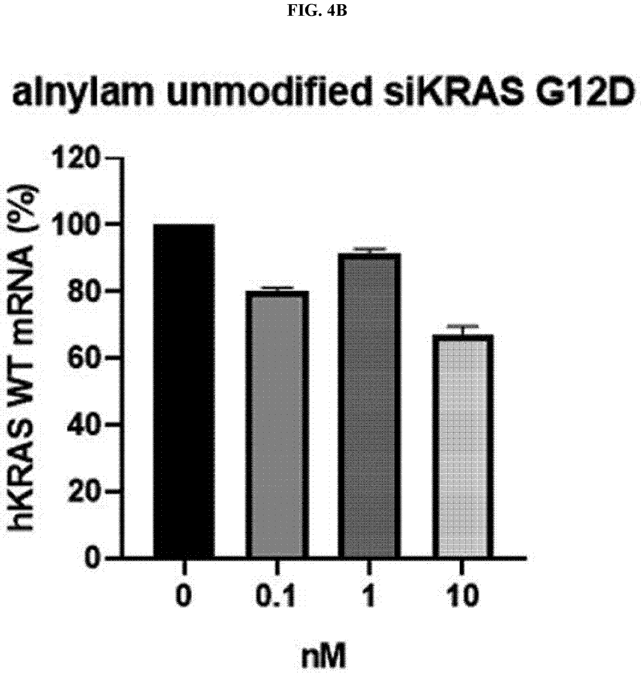

FIG. 4A shows the inhibitory activity on KRAS mRNA expression when some of the 29 lead siRNAs of the present invention were treated on a pancreatic cancer cell line, and FIG. 4B shows the experimental results for the control group.

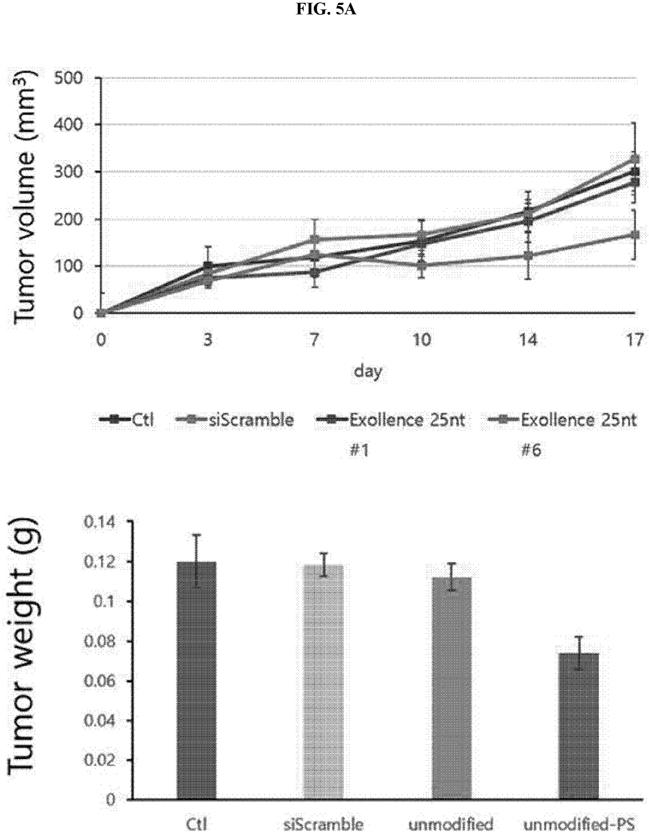

FIG.5A shows the effects on reducing tumor size and tumor weight when some of the 29 lead siRNAs of the present invention were administered to a pancreatic cancer animal model(tumor volume: in order from the highest point of the graph, siScramble, Ctl, Exollence 25nt #1, and Exollence 25nt #6).

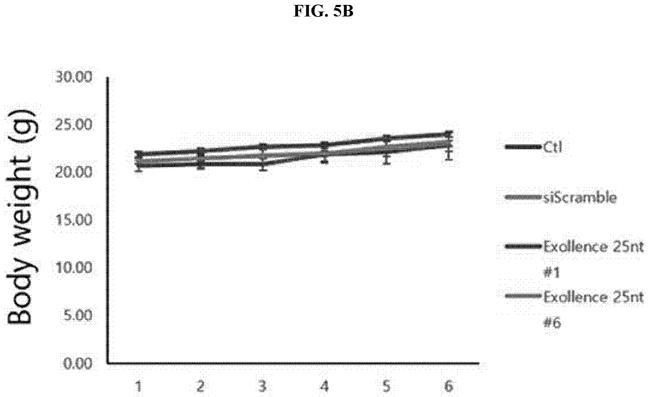

FIG. 5B shows the experimental results for changes in animal weight when some of the 29 lead siRNAs of the present invention were administered to a pancreatic cancer animal model (in order from the highest point of the graph, Exollence 25nt #1, Exollence 25nt #6, siScramble, and Ctl).

[Modes of the Invention]

The inventors of the present invention produced novel siRNAs by introducing various modifications into siRNA, and as a result of treating these siRNAs on pancreatic cancer or colorectal cancer cell lines, discovered 29 siRNAs that exhibited excellent inhibitory activity against KRAS G12D and corresponding anticancer effects. In particular, the 29 siRNAs of the present invention comprise a sense strand and an anti-sense strand, and the sense strand and anti-sense strand of each siRNA have different characteristics in terms of length, chemical modification, and sequence modification. Therefore, the inventors confirmed that each siRNA exhibits the colorectal cancer and pancreatic cancer therapeutic activity identified in the present invention by possessing both the sequence characteristics of the sense strand and the anti-sense strand, thereby completing the present invention.

Therefore, the present invention provides a pharmaceutical composition for preventing or treating cancer, comprising one or more selected from the group consisting of siRNAs having any one nucleotide sequence selected from the group consisting of SEQ ID NOs: 1 to 29 as an active ingredient.

Additionally, the present invention provides a pharmaceutical composition for preventing or treating cancer, comprising one or more selected from the group consisting of siRNAs having any one nucleotide sequence selected from the group consisting of SEQ ID NOs: 30 to 58 as an active ingredient.

In the present invention, the siRNAs (ss strand) comprising any one nucleotide sequence selected from the group consisting of SEQ ID NOs: 1 to 29 are as follows (Table 3):

1) unmodified siKRAS G12D represented by SEQ ID NO: 1;
2) unmodified siKRAS G12D-2 represented by SEQ ID NO: 2;
3) unmodified siKRAS G12D-PS represented by SEQ ID NO: 3;
4) unmodified siKRAS G12D-PS-2 represented by SEQ ID NO: 4;
5) Pattern_unmodified siKRAS G12D represented by SEQ ID NO: 5;
6) Pattern_OME modified siKRAS G12D represented by SEQ ID NO: 6;
7) Exollence_25nt #3 represented by SEQ ID NO: 7;
8) Exollence_25nt #4 represented by SEQ ID NO: 8;
9) Exollence_25nt #5 represented by SEQ ID NO: 9;
10) Exollence_25nt #6 represented by SEQ ID NO: 10;
11) Exollence_25nt #7 represented by SEQ ID NO: 11;
12) Exollence_25nt #8 represented by SEQ ID NO: 12;
13) Exollence_25nt #9 represented by SEQ ID NO: 13;
14) Exollence_25nt-#12 represented by SEQ ID NO: 14;

15) Exollence_25nt #13 represented by SEQ ID NO: 15;
16) Exollence_25nt #16 represented by SEQ ID NO: 16;
17) Exollence_25nt #28 represented by SEQ ID NO: 17;
18) Exollence_25nt #29 represented by SEQ ID NO: 18;
19) Exollence_25nt #30 represented by SEQ ID NO: 19;
20) Exollence_25nt #32 represented by SEQ ID NO: 20;
21) Exollence_25nt #33 represented by SEQ ID NO: 21;
22) Exollence_25nt #34 represented by SEQ ID NO: 22;
23) Exollence_25nt #36 represented by SEQ ID NO: 23;
24) Exollence_25nt #37 represented by SEQ ID NO: 24;
25) Exollence_25nt #38 represented by SEQ ID NO: 25;
26) Exollence_25nt #40 represented by SEQ ID NO: 26;
27) Exollence_25nt #41 represented by SEQ ID NO: 27;
28) Exollence_25nt #42 represented by SEQ ID NO: 28; and
29) Exollence_25nt #44 represented by SEQ ID NO: 29.

Additionally, in the present invention, the siRNAs (as strand) comprising any one nucleotide sequence selected from the group consisting of SEQ ID NOs: 30 to 58 are as follows (Table 4):

1) unmodified siKRAS G12D represented by SEQ ID NO: 30;
2) unmodified siKRAS G12D-2 represented by SEQ ID NO: 31;
3) unmodified siKRAS G12D-PS represented by SEQ ID NO: 32;
4) unmodified siKRAS G12D-PS-2 represented by SEQ ID NO: 33;
5) Pattern_unmodified siKRAS G12D represented by SEQ ID NO: 34;
6) Pattern_OME modified siKRAS G12D represented by SEQ ID NO: 35;
7) Exollence_25nt #3 represented by SEQ ID NO: 36;
8) Exollence_25nt #4 represented by SEQ ID NO: 37;
9) Exollence_25nt #5 represented by SEQ ID NO: 38;
10) Exollence_25nt #6 represented by SEQ ID NO: 39;
11) Exollence_25nt #7 represented by SEQ ID NO: 40;
12) Exollence_25nt #8 represented by SEQ ID NO: 41;
13) Exollence_25nt #9 represented by SEQ ID NO: 42;
14) Exollence_25nt-#12 represented by SEQ ID NO: 43;
15) Exollence_25nt #13 represented by SEQ ID NO: 44;
16) Exollence_25nt #16 represented by SEQ ID NO: 45;
17) Exollence_25nt #28 represented by SEQ ID NO: 46;
18) Exollence_25nt #29 represented by SEQ ID NO: 47;
19) Exollence_25nt #30 represented by SEQ ID NO: 48;
20) Exollence_25nt #32 represented by SEQ ID NO: 49;
21) Exollence_25nt #33 represented by SEQ ID NO: 50;
22) Exollence_25nt #34 represented by SEQ ID NO: 51;
23) Exollence_25nt #36 represented by SEQ ID NO: 52;
24) Exollence_25nt #37 represented by SEQ ID NO: 53;
25) Exollence_25nt #38 represented by SEQ ID NO: 54;
26) Exollence_25nt #40 represented by SEQ ID NO: 55;
27) Exollence_25nt #41 represented by SEQ ID NO: 56;
28) Exollence_25nt #42 represented by SEQ ID NO: 57; and
29) Exollence_25nt #44 represented by SEQ ID NO: 58.

In the present invention, 29 siRNAs were produced by applying modifications such as chemical modifications, adjustment of sequence length, and sequence shifting based on KRAS G12D siRNA.
At this time, SEQ ID NOs: 1 to 29 of the present invention are the sense strand sequences of each siRNA. Additionally, SEQ ID NOs: 30 to 58 are the antisense strand sequences of the siRNAs. Therefore, the present invention provides a pharmaceutical composition for preventing or treating cancer, comprising one or more selected from the group consisting of siRNAs, each comprising a sense strand represented by any one nucleotide sequence selected from the group consisting of SEQ ID NOs: 1 to 29 and an antisense strand comprising a complementary sequence represented by any one nucleotide sequence selected from the group consisting of SEQ ID NOs: 30 to 58, as an active ingredient.
In the present invention, the term "sense strand" refers to a polynucleotide having a nucleotide sequence identical to a target nucleic acid, which means a polynucleotide that is wholly or partially identical to an mRNA, a non-mRNA RNA sequence, or a coding or non-coding DNA sequence. Additionally, the term "antisense strand" refers to a polynucleotide that is substantially or 100% complementary to a target nucleic acid, and generally refers to RNA having a nucleotide sequence complementary to the nucleotide sequence of the sense strand. For example, it may be wholly or partially complementary to an mRNA (messenger RNA), a non-mRNA RNA sequence (microRNA, piwiRNA, tRNA, rRNA, and hnRNA), or a coding or non-coding DNA sequence.

Therefore, in the present invention, an siRNA defined by its sense strand may be understood to form a double strand with an siRNA defined by its corresponding antisense strand, and since siRNA is a double strand, it may be understood to include both the sense strand and the antisense strand. For example, when referring to any one siRNA selected from the group consisting of SEQ ID NOs: 1 to 29 based on the sense strand in the present invention, it may be understood that the siRNA represented by SEQ ID NO: 1 has an antisense strand corresponding to a nucleotide sequence represented by SEQ ID NO: 30.

Therefore, the present invention provides siRNAs comprising a sense strand represented by any one nucleotide sequence selected from the group consisting of SEQ ID NOs: 1 to 29 and an antisense strand represented by any one nucleotide sequence selected from the group consisting of SEQ ID NOs: 30 to 58, as follows:

1) unmodified siKRAS G12D comprising a sense strand represented by SEQ ID NO: 1 and an antisense strand represented by SEQ ID NO: 30;
2) unmodified siKRAS G12D-2 comprising a sense strand represented by SEQ ID NO: 2 and an antisense strand represented by SEQ ID NO: 31;
3) unmodified siKRAS G12D-PS comprising a sense strand represented by SEQ ID NO: 3 and an antisense strand represented by SEQ ID NO: 32;
4) unmodified siKRAS G12D-PS-2 comprising a sense strand represented by SEQ ID NO: 4 and an antisense strand represented by SEQ ID NO: 33;
5) Pattern_unmodified siKRAS G12D comprising a sense strand represented by SEQ ID NO: 5 and an antisense strand represented by SEQ ID NO: 34;
6) Pattern_OME modified siKRAS G12D comprising a sense strand represented by SEQ ID NO: 6 and an antisense strand represented by SEQ ID NO: 35;
7) Exollence_25nt #3 comprising a sense strand represented by SEQ ID NO: 7 and an antisense strand represented by SEQ ID NO: 36;
8) Exollence_25nt #4 comprising a sense strand represented by SEQ ID NO: 8 and an antisense strand represented by SEQ ID NO: 37;
9) Exollence_25nt #5 comprising a sense strand represented by SEQ ID NO: 9 and an antisense strand represented by SEQ ID NO: 38;
10) Exollence_25nt #6 comprising a sense strand represented by SEQ ID NO: 10 and an antisense strand represented by SEQ ID NO: 39;
11) Exollence_25nt #7 comprising a sense strand represented by SEQ ID NO: 11 and an antisense strand represented by SEQ ID NO: 40;
12) Exollence_25nt #8 comprising a sense strand represented by SEQ ID NO: 12 and an antisense strand represented by SEQ ID NO: 41;
13) Exollence_25nt #9 comprising a sense strand represented by SEQ ID NO: 13 and an antisense strand represented by SEQ ID NO: 42;
14) Exollence_25nt-#12 comprising a sense strand represented by SEQ ID NO: 14 and an antisense strand represented by SEQ ID NO: 43;
15) Exollence_25nt #13 comprising a sense strand represented by SEQ ID NO: 15 and an antisense strand represented by SEQ ID NO: 44;
16) Exollence_25nt #16 comprising a sense strand represented by SEQ ID NO: 16 and an antisense strand represented by SEQ ID NO: 45;
17) Exollence_25nt #28 comprising a sense strand represented by SEQ ID NO: 17 and an antisense strand represented by SEQ ID NO: 46;
18) Exollence_25nt #29 comprising a sense strand represented by SEQ ID NO: 18 and an antisense strand represented by SEQ ID NO: 47;
19) Exollence_25nt #30 comprising a sense strand represented by SEQ ID NO: 19 and an antisense strand represented by SEQ ID NO: 48;
20) Exollence_25nt #32 comprising a sense strand represented by SEQ ID NO: 20 and an antisense strand represented by SEQ ID NO: 49;
21) Exollence_25nt #33 comprising a sense strand represented by SEQ ID NO: 21 and an antisense strand represented by SEQ ID NO: 50;

22) Exollence_25nt #34 comprising a sense strand represented by SEQ ID NO: 22 and an antisense strand represented by SEQ ID NO: 51;

23) Exollence_25nt #36 comprising a sense strand represented by SEQ ID NO: 23 and an antisense strand represented by SEQ ID NO: 52;

24) Exollence_25nt #37 comprising a sense strand represented by SEQ ID NO: 24 and an antisense strand represented by SEQ ID NO: 53;

25) Exollence_25nt #38 comprising a sense strand represented by SEQ ID NO: 25 and an antisense strand represented by SEQ ID NO: 54;

26) Exollence_25nt #40 comprising a sense strand represented by SEQ ID NO: 26 and an antisense strand represented by SEQ ID NO: 55;

27) Exollence_25nt #41 comprising a sense strand represented by SEQ ID NO: 27 and an antisense strand represented by SEQ ID NO: 56;

28) Exollence_25nt #42 comprising a sense strand represented by SEQ ID NO: 28 and an antisense strand represented by SEQ ID NO: 57; and

29) Exollence_25nt #44 comprising a sense strand represented by SEQ ID NO: 29 and an antisense strand represented by SEQ ID NO: 58.

The technology for suppressing gene expression is an important tool for developing therapeutics and verifying targets for disease treatment. Among these technologies, RNA interference (hereinafter referred to as "RNAi") was found to function in this role, and it has been revealed that RNAi acts on sequence-specific mRNA in various types of mammalian cells. When a long double-stranded RNA is delivered into a cell, the delivered RNA double strand is converted by an endonuclease called Dicer into a short interfering RNA (small interfering RNA, hereinafter referred to as "siRNA") of 21 to 23 base pairs (bp), and the siRNA binds to the RISC (RNA-induced silencing complex) to inhibit the expression of the target gene in a sequence-specific manner through a process in which the guide (antisense) strand recognizes and degrades the target mRNA.

That is, in the present invention, "siRNA (small interfering RNA)" refers to a small RNA fragment of 18 to 23 nucleotides in size, which is produced by cleavage of a double-stranded RNA by a Dicer enzyme, and may be used to suppress the expression of a corresponding protein or mRNA by specifically binding to an mRNA having a complementary sequence.

Additionally, the 29 siRNAs of the present invention may be delivered to a subject in a form comprising a nucleic acid delivery system.

In the present invention, the term "nucleic acid delivery system" refers to a system for enhancing delivery efficiency into the body, which has the advantages of excellent stability in the body and a simple manufacturing process as a drug.

The nucleic acid delivery system may include, but is not limited to, viral vectors, non-viral vectors, liposomes, cationic polymers, micelles, emulsions, and solid lipid nanoparticles.

That is, in the present invention, the siRNA nucleic acid molecule may be provided in a form included in an expression vector. The expression vector may preferably encode the miRNA of the present invention in an expressible form, and may be introduced into a host by methods generally used in the art, and in this case, the expression vector in the present invention may be used without limitation as long as it is for delivering siRNA. Additionally, in the present invention, the expression vector may preferably further include a selection marker to facilitate selection of transformed cells, but is not limited thereto. In the present invention, the expression vector may be introduced into a host cell and provided as a transformed transformant, but is not limited thereto.

Viral vectors have advantages of high delivery efficiency and long duration, and include retroviral vectors, adenoviral vectors, vaccinia virus vectors, adeno-associated viral vectors, and oncolytic viral vectors. Nonviral vectors may include plasmids. In addition, various formulations such as liposomes, cationic polymers, micelles, emulsions, and solid lipid nanoparticles may be used. Cationic polymers for nucleic acid delivery may include natural polymers such as chitosan, atelocollagen, and cationic polypeptides, as well as synthetic polymers such as poly(L-lysine), linear or branched polyethylene imine (PEI), cyclodextrin-based polycations, and dendrimers.

When the siRNA is included in the composition in the form of a complex with the nucleic acid delivery system as described above, it may efficiently promote delivery of the siRNA to target cells, so that even with a relatively low dose, it is delivered to the target cells and exhibits a high target gene expression regulation function. Additionally, there is an advantage in that it may prevent non-specific delivery of the siRNA to organs and cells other than the target.

In the present invention, the siRNA may be chemically modified. Such chemical modifications of the siRNA may be for improving in vivo stability, imparting nuclease resistance, and reducing non-specific immune responses, but are not limited thereto.

In the present invention, it was confirmed that all of the 29 siRNAs exhibited anticancer activity against colorectal cancer or pancreatic cancer, and as a result of analyzing common features in the sequences of the 29 siRNAs, it was confirmed that they are classified into three groups.

First, the first group and common features are as follows. In one embodiment of the present invention, the group consisting of the following siRNAs has a sense strand that is 19 to 25 nt long and an anti-sense strand that is 21 to 27 nt long, but is not limited thereto:

1) siRNA comprising any one nucleotide sequence selected from the group consisting of SEQ ID NOs: 1 to 5;
2) siRNA comprising a nucleotide sequence represented by SEQ ID NO: 7;
3) siRNA comprising any one nucleotide sequence selected from the group consisting of SEQ ID NOs: 14, 15, and 17; and
4) siRNA comprising any one nucleotide sequence selected from the group consisting of SEQ ID NOs: 20, 23, and 26 to 28.

The second group and common features are as follows. In one embodiment of the present invention, the group consisting of siRNA comprising a nucleotide sequence represented by SEQ ID NO: 12, siRNA comprising a nucleotide sequence represented by SEQ ID NO: 13, siRNA comprising a nucleotide sequence represented by SEQ ID NO: 18, siRNA comprising a nucleotide sequence represented by SEQ ID NO: 21, siRNA comprising a nucleotide sequence represented by SEQ ID NO: 24, and siRNA comprising a nucleotide sequence represented by SEQ ID NO: 29 may be characterized by the following features, but is not limited thereto:

a) having a sense strand that is 19 to 25 nt long and an anti-sense strand that is 21 to 27 nt long;
b) having no tt overhang in a sense strand;
c) having a 2'-OMe-chemically modified nucleotide at the 7th position from the 5' end of a sense strand, and a nucleotide in an anti-sense strand binding to this position, which is not chemically modified with 2'-OMe;
d) having a nucleotide at the 9th position from the 5' end of the sense strand, which is not chemically modified with 2'-OMe; and
e) having 2'-OMe-chemically modified nucleotides at the 18th and 20th positions from the 5' end of the anti-sense strand.

Lastly, the third group and common features are as follows. In one embodiment of the present invention, the group consisting of siRNA comprising a nucleotide sequence represented by SEQ ID NO: 6, siRNA comprising a nucleotide sequence represented by SEQ ID NO: 8, siRNA comprising a nucleotide sequence represented by SEQ ID NO: 9, siRNA comprising a nucleotide sequence represented by SEQ ID NO: 10, siRNA comprising a nucleotide sequence represented by SEQ ID NO: 11, siRNA comprising a nucleotide sequence represented by SEQ ID NO: 16, siRNA comprising a nucleotide sequence represented by SEQ ID NO: 19, siRNA comprising a nucleotide sequence represented by SEQ ID NO: 22, and siRNA comprising a nucleotide sequence represented by SEQ ID NO: 25 may be characterized by the following features, but is not limited thereto:

a) having a sense strand that is 19 to 25 nt long and an anti-sense strand that is 21 to 27 nt long;
b) having a 2'-OMe-chemically modified nucleotide at the 7th position from the 5' end of a sense strand, and a nucleotide in an anti-sense strand binding to this position, which is not chemically modified with 2'-OMe;
c) having a nucleotide at the 9th position from the 5' end of the sense strand, which is not chemically modified with 2'-OMe; and
d) having 2'-OMe-chemically modified nucleotides at the 16th, 18th and 20th positions from the 5' end of the anti-sense strand.

In the present invention, the term "overhang" may refer to an unpaired nucleotide at the end of a DNA or RNA molecule. Such unpaired nucleotides may be present on any strand and may create a 3' or 5' overhang. Longer overhangs may be referred to as cohesive ends or sticky ends, but are not limited thereto. The 29 siRNAs of the present invention were shown to be classified into three groups depending on whether overhangs are present in parts of the sense strand or the anti-sense strand. Therefore, in the present invention, it may be understood that the therapeutic effect of the siRNA on colorectal cancer or pancreatic cancer is exhibited depending on the presence of overhangs, whether they are present on the sense strand or the anti-sense strand, and the type of overhang.

In the present invention, "-OME" may refer to a methoxy group and may indicate an effect of being chemically modified by being linked to a nucleotide. The chemical modification may exhibit different chemical characteristics depending on the type or sequence of nucleotides, and furthermore, may produce different effects on colorectal cancer or pancreatic cancer therapeutic activity. Therefore, it is apparent that the 29 siRNAs of the present invention exhibit unique colorectal cancer or pancreatic cancer therapeutic activity by having chemical modifications applied to any one of the sense strand or the anti-sense strand, at specific positions, or to specific types of nucleotides. Such specificity and uniqueness of the modifications may be understood to apply equally not only to the chemical modifications but also to sequence modifications applied to the nucleotide sequences of the siRNA. In conclusion, the 29 siRNAs of the present invention correspond to siRNAs that exhibit therapeutic effects on colorectal cancer or pancreatic cancer among an extremely large number of possible siRNAs, by having sequence length adjustments, chemical modifications, and sequence modifications applied respectively to any one or more of the sense strand or the anti-sense strand.

In the present invention, it was confirmed that when each of the 29 siRNAs, in particular, the siRNAs represented by SEQ ID NOs: 1 to 3 or SEQ ID NO: 6, was administered, the expression of KRAS mRNA was inhibited. Therefore, the siRNA of the present invention may be applied to cancers that may be treated by inhibiting the expression of KRAS mRNA. In one embodiment of the present invention, the cancer may be one or more selected from the group consisting of pancreatic cancer, colorectal cancer, squamous cell carcinoma, lung cancer, adenocarcinoma of the lung, peritoneal cancer, skin cancer, skin or intraocular melanoma, rectal cancer, anal cancer, esophageal cancer, small intestinal cancer, endocrine cancer, parathyroid cancer, adrenal cancer, soft tissue sarcoma, urethral cancer, blood cancer, liver cancer, gastric cancer, glioblastoma, cervical cancer, ovarian cancer, bladder cancer, liver tumors, breast cancer, colon cancer, endometrial cancer, uterine cancer, salivary gland cancer, kidney cancer, prostate cancer, vulvar cancer, thyroid cancer, head and neck cancer, and brain cancer, but is not limited thereto.

In one embodiment of the present invention, the composition may inhibit the proliferation of cancer cells, but is not limited thereto.

In one embodiment of the present invention, the composition may reduce tumor size and weight, but is not limited thereto.

KRAS is one of the oncogenes that is frequently mutated in human tumors. Normal KRAS performs essential functions in signal transduction in normal tissues, but mutations of the KRAS gene are involved in the development of various cancers, and therefore, mutations of the KRAS gene are known as important therapeutic targets.

Mutations of the KRAS gene usually occur at codons, and mutations are generally frequent at codons 12, 13, and 61. In particular, mutations occur most frequently at codon 12, and among them, G12C is known to account for approximately 40% of all KRAS mutant genes.

In one embodiment of the present invention, the composition may inhibit KRAS mRNA expression, but is not limited thereto.

In the present invention, it was confirmed that the siRNA produced in the present invention exhibited a remarkable expression inhibitory effect on KRAS G12D among KRAS. Therefore, it was demonstrated that the siRNA of the present invention, by inhibiting KRAS G12D, exhibited excellent preventive or therapeutic effects on pancreatic cancer and colorectal cancer, which are known to be highly associated with KRAS G12D.

The patient to whom the composition of the present invention is administered may be a mammal, preferably a human, monkey, or rodent (mouse, rat), and particularly may be any mammal having pancreatic cancer or colorectal cancer, for example, a human.

In order to increase the therapeutic effect on pancreatic cancer or colorectal cancer, the concentration of the siRNA in the composition, or the use or treatment concentration, may be 0.001 to 1000 nM, preferably 0.01 to 100 nM, and more preferably 0.1 to 10 nM, but is not limited thereto.

The composition of the present invention, which includes an siRNA comprising a nucleotide sequence represented by SEQ ID NOs: 1 to 29, may achieve a synergistic effect through combination therapy.

The composition of the present invention may be prepared to additionally include at least one pharmaceutically acceptable carrier besides the above active ingredient. The pharmaceutically acceptable carrier must be compatible with the active ingredient of the present invention and may include saline, sterile water, Ringer's solution, buffered saline, dextrose solution, maltodextrin solution, glycerol, ethanol, and one or more mixtures of these components, and if necessary, other conventional additives such as antioxidants, buffers, and bacteriostats may be added. Additionally, diluents, dispersing agents, surfactants, binders, and lubricants may be further added to formulate an injectable preparation such as an aqueous solution, suspension, or emulsion. Furthermore, it may be desirably formulated according to each disease or ingredient by an appropriate method in the art or by a method disclosed in Remington's Pharmaceutical Science (Mack Publishing Company, Easton PA).

The content of the active ingredient included in the composition of the present invention and the method of administration may be determined by those of ordinary skill in the art based on the symptoms of a typical patient and the severity of the disease. Additionally, it may be formulated in various forms such as powders, tablets, capsules, liquids, injections, ointments, and syrups, and may also be provided in unit-dose or multi-dose containers, such as sealed ampoules and vials.

The composition of the present invention may be administered orally or parenterally. The administration route of the composition according to the present invention is not limited thereto, but may be, for example, oral, intravenous, intramuscular, intra-arterial, intramedullary, intradural, intracardiac, transdermal, subcutaneous, intraperitoneal, enteral, sublingual, or topical administration. The dosage of the composition according to the present invention may vary depending on the patient's body weight, age, gender, health condition, diet, time and method of administration, excretion rate, or severity of the disease, and may be easily determined by those of ordinary skill in the art. Additionally, for clinical administration, the composition of the present invention may be formulated into a suitable dosage form using known techniques.

The siRNA of the present invention may be introduced into cells in vivo or ex vivo for the treatment of cancer. In this way, by combining the siRNA of the present invention with chemotherapy to increase sensitivity to chemotherapeutic agents, therapeutic effects may be maximized and side effects may be reduced.

Additionally, as used herein in connection with amino acid sequences and/or nucleic acid sequences, the terms "percent identity," "sequence identity," "percent similarity," "sequence similarity," and "percent sequence identity" may refer to a measure determined by comparing the degree of similarity between two sequences based on an alignment of the sequences that maximizes similarity among aligned amino acid residues or nucleotides, taking into account the number of identical or similar residues or nucleotides, the total number of residues or nucleotides, and the presence and length of gaps in the sequence alignment, but is not limited thereto.

Portions of the polynucleotide or polypeptide sequences may include insertions or deletions (i.e., gaps) relative to a reference sequence (which does not include insertions or deletions) for optimal alignment of the two sequences. The above percentage is calculated by determining the number of positions where identical nucleotide bases or amino acid residues occur in both sequences to obtain the number of matching positions, dividing the number of matching positions by the total number of positions in the comparison window, and multiplying the result by 100 to yield the percent sequence identity.

With respect to two or more nucleic acid or polypeptide sequences, the term "identical" or percentage "identity" refers to two or more sequences or subsequences having a specified percentage of identical amino acid residues or nucleotides (i.e., about 60% identity, preferably 65%, 70%, 75%, 80%, 85%, 90%, 91%, 92%, 93%, 94%, 95%, 96%, 97%, 98%, 99% or higher identity for the specified region when compared and aligned for maximum correspondence over a comparison window or displayed region), as measured using the BLAST or BLAST 2.0 sequence comparison algorithms employing default parameters described below, or by manual alignment and visual inspection (see, for example, the NCBI website http://www.ncbi.nlm.nih.gov/BLAST/). Subsequently, such sequences are referred to as being "substantially identical."

This definition may also refer to, or be applied to, the complement of a test sequence. The above definition also encompasses sequences having deletions and/or insertions, as well as substitutions. As described below, preferred algorithms may account for gaps and the like. Preferably, the identity exists over a region of at least about 25 amino acids or nucleotides in length, or more preferably over a region of 50 to 100 amino acids or nucleotides in length.

The "position" of an amino acid or nucleotide base is indicated by a number that sequentially identifies each amino acid (or nucleotide base) in the reference sequence based on its location relative to the N-terminus (or 5'-end). Due to deletions, insertions, truncations, fusions, and the like that must be considered in determining optimal alignment, the number of amino acid residues in a test sequence as determined simply by counting from the N-terminus generally will not necessarily be the same as the number of corresponding positions in the reference sequence. For example, if a variant has a deletion relative to the aligned reference sequence, there will be no amino acid in the variant corresponding to the position within the deletion in the reference sequence. If there is an insertion in the aligned reference sequence, the insertion will not correspond to a numbered amino acid position in the reference sequence. In cases of truncation or fusion, there may be extensions of amino acids in one of the reference or aligned sequences that do not correspond to any amino acid in the other sequence.

When used in connection with the numbering of a given amino acid or polynucleotide sequence, the terms "numbered with respect to" or "corresponding to" refer to the numbering of residues in a specified reference sequence when the given amino acid or polynucleotide sequence is compared to the reference sequence.

As used herein, the term "active ingredient" refers to a substance or group of substances in which the pharmacological action of the ingredient itself, directly or indirectly, is expected to produce the efficacy and effects of the preparation, meaning an ingredient that exhibits the intended activity alone or may exhibit the intended activity together with a carrier that itself does not have activity. The ingredient may be included in an amount of at least 1% by weight based on the dry weight of the total composition, and for example, may be included in an amount of 10% to 100% by weight, 20% to 100% by weight, 30% to 100% by weight, 40% to 100% by weight, 50% to 100% by weight, 60% to 100% by weight, 70% to 100% by weight, 80% to 100% by weight, 90% to 100% by weight, 10% to 80% by weight, 20% to 80% by weight, 30% to 80% by weight, 40% to 80% by weight, 50% to 80% by weight, 60% to 80% by weight, or 70% to 80% by weight, but is not limited thereto.

As described above, in the present invention, it was confirmed that when the 29 siRNAs of the present invention were administered, excellent therapeutic activity against colorectal cancer or pancreatic cancer was exhibited, and thus, the composition of the present invention may have any one or more of the 29 siRNAs and a nucleic acid delivery system as active ingredients.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and *Primojel*®; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite ($NaHSO_3$) carbon dioxide gas, sodium metabisulfite ($Na_2S_2O_5$), sodium sulfite ($Na_2SO_3$), nitrogen gas ($N_2$), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases. Specifically, the effective amount of the composition according to the present invention may vary depending on the age, gender, and weight of the patient, and generally may be administered at 0.001 to 150 mg, preferably 0.01 to 100 mg, per kg of body weight daily or every other day, or divided into 1 to 3 administrations per day. However, the above dosage may be increased or decreased depending on the administration route, severity of the disease, gender, body weight, age, and the like, and therefore does not limit the scope of the present invention in any way.

As used herein, the "subject" refers to a subject in need of treatment of a disease, and more specifically, refers to a mammal such as a human or a non-human primate, a mouse, a rat, a dog, a cat, a horse, and a cow.

As used herein, the "administration" refers to providing a subject with a predetermined composition of the present invention by using an arbitrary appropriate method. The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

The present invention provides a kit for preventing or treating cancer, including a composition comprising one or more selected from the group consisting of siRNAs having any one nucleotide sequence selected from the group consisting of SEQ ID NOs: 1 to 29 as an active ingredient, and an instruction manual.

The "kit" of the present invention may further include other components, devices, or substances that are conventionally required for treating cancer, in addition to the above-described components. Additionally, all components included in the kit may be used one or more times without limitation on the number of uses, there is no restriction on the order in which each substance is used, and the application of each substance may proceed simultaneously or sequentially.

In the present invention, the kit may include a container, instructions, and the like. The container may serve to package the above substance, and may also serve to store and secure it. The material of the container may take the form of, for example, a bottle, tub, sachet, envelope, tube, or ampoule, and may be formed partially or entirely from plastic, glass, paper, foil, wax, or the like. The container may be equipped with a cap that is initially a part of the container or is completely or partially detachable and attachable to the container by mechanical, adhesive, or other means, and may also be equipped with a stopper that allows access to the contents with a syringe needle. The kit may include an external package, and the external package may include instructions for use of the components, but is not limited thereto.

Hereinafter, preferred examples are presented to help understand the present invention. However, the following examples are provided only to help understand the present invention more easily, and the contents of the present invention are not limited by the following examples.

[Examples]

**Example 1. Selection of siRNA candidates for cancer treatment and confirmation of lead substances**

Example 1-1. Selection of siRNA candidates for cancer treatment

To find an RNA agent with enhanced gene-silencing effects, siRNA having an RNA sequence with a chemical modification introduced to the sequence was obtained based on KRAS G12D siRNA. Here, the reference RNA sequence without modification is as follows.

[Table 1]

| No. | Name | Sequence |
|-----|------|----------|
| 59 | base RNA_SS | GUU GGA GCU GAU GGC GUA G=tt |
| 60 | base RNA_AS | CUA CGC CAU CAG CUC CAA C=tt |

Specifically, test parameters for siRNA modification conditions for effective candidate material selection are as follows.

[Table 2]

| No. | Modification | Content |
|-----|-------------|---------|
| a | siRNA length (nt) | 19 ~ 25 nt |
| b | Presence of tt overhang in SS | (-) |
| c | Presence of tt overhang in AS | (+) |
| d | Presence of phosphorothioate (PS) modification in tt overhang | (+) |
| e | Type of nucleotides chemical modification* | 2'-OME |
| f | Number of modified nucleotides (Pattern) | 0, 3, 4 |

(continued)

| No. | Modification | Content |
|---|---|---|
| g | Sequence of the 20th nucleotide | When G/C, modified into A/U |

(a. the sense strand is 19 to 25 nt long, the anti-sense strand is 21 to 27 nt long b. the sense strand has no tt overhang c. the anti-sense strand has a tt overhang d. PS modification at the first t of the 5' end in the tt overhang present in the anti-sense strand e. chemical modification made by 2'-OMe method f-1. a 2'-OMe-chemically-modified nucleotide at the 7th position from the 5' end of the sense strand (no nucleotide modification in an anti-sense strand binding to this position) f-2. the nucleotide at the 9th position from the 5' end of the sense strand is not modified f-3. the nucleotides at the 16th, 18th, and 20th positions from the 5' end of the anti-sense strand are not chemically modified with 2'-OMe f-3-1. not modified f-3-2. both 18th and 20th nucleotides are modified

f-3-3. all of 16th, 18th and 20th nucleotides are modified

*f-1 and f-2 are mandatory conditions, and f-3 has three cases depending on the number of modifications

g-1. the 20th nucleotide from the 5' end of the sense strand is substituted with A and the 6th nucleotide from the 5' end of the anti-sense strand is substituted with U

g-2. the 20th nucleotide from the 5' end of the sense strand is substituted with U and the 6th nucleotide from the 5' end of the anti-sense strand is substituted with A indicates the case of g-1 or g-2, but not including both conditions)

As a result, finally, 105 siRNA candidate materials were selected.

Example 1-2. Identification of siRNA lead substances for cancer treatment

To identify a lead substance from the 105 types of siRNAs selected in Example 1-1, the survival rate of cancer cells when treating the cancer cells was confirmed, and to this end, apoptotic effects were analyzed. Specifically, the cell survival rates obtained after treating three types of pancreatic cancer cell lines and three types of colorectal cancer cell lines with the 105 types of siRNAs were summarized, and present in one graph (analyzing a total of 18 values) according to the ranking. Here, the obtained value is n=3 per cell line.

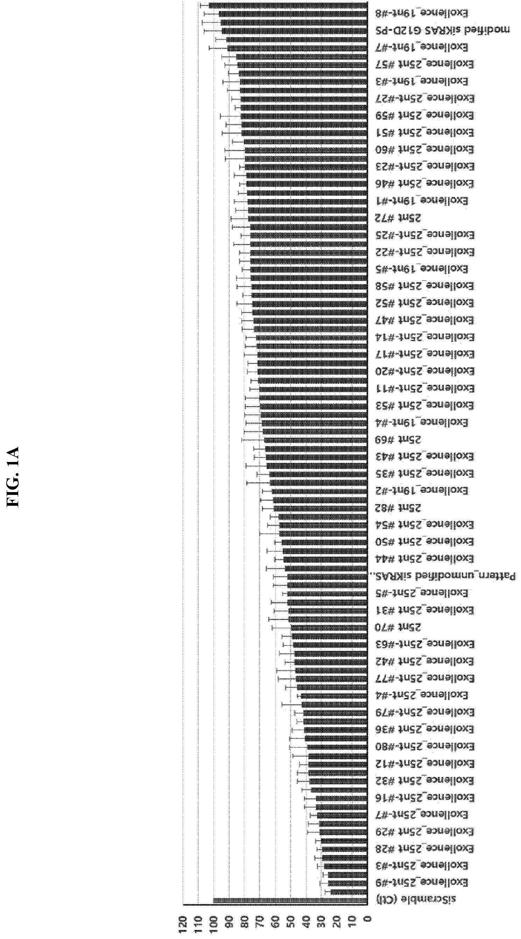

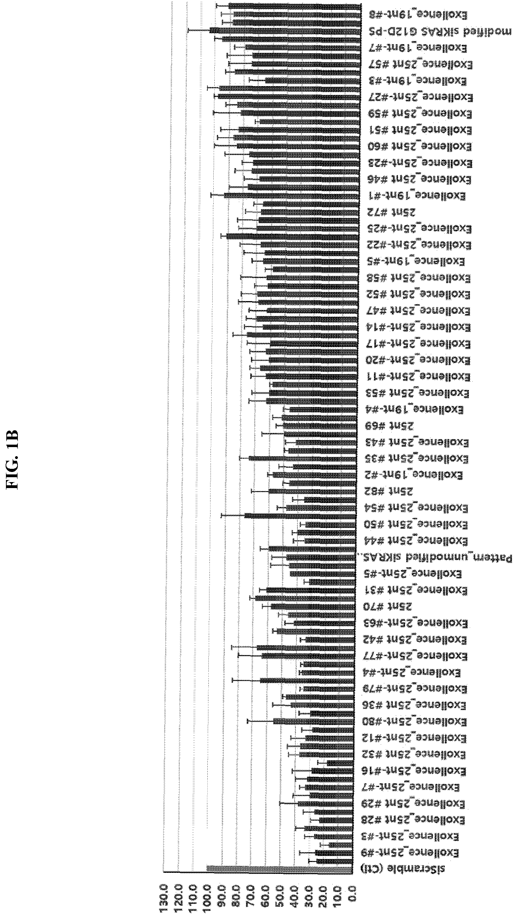

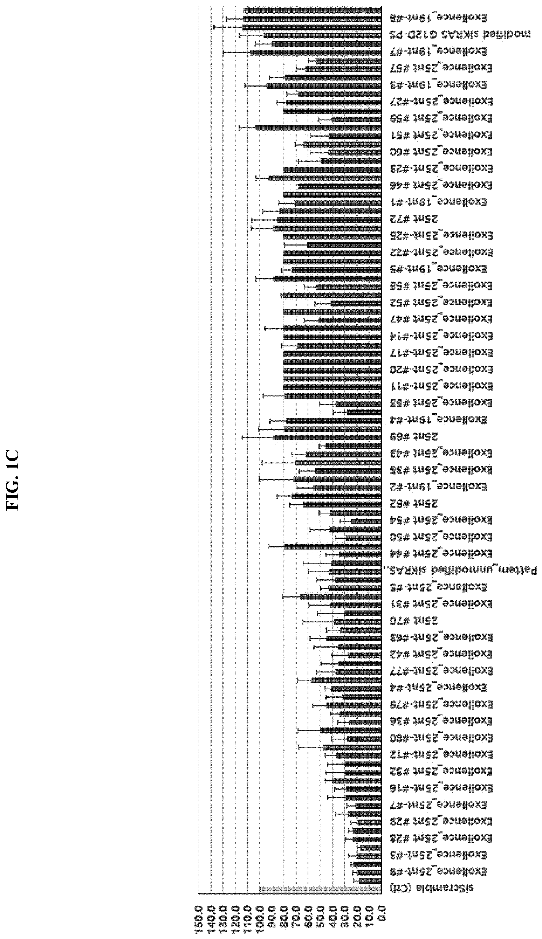

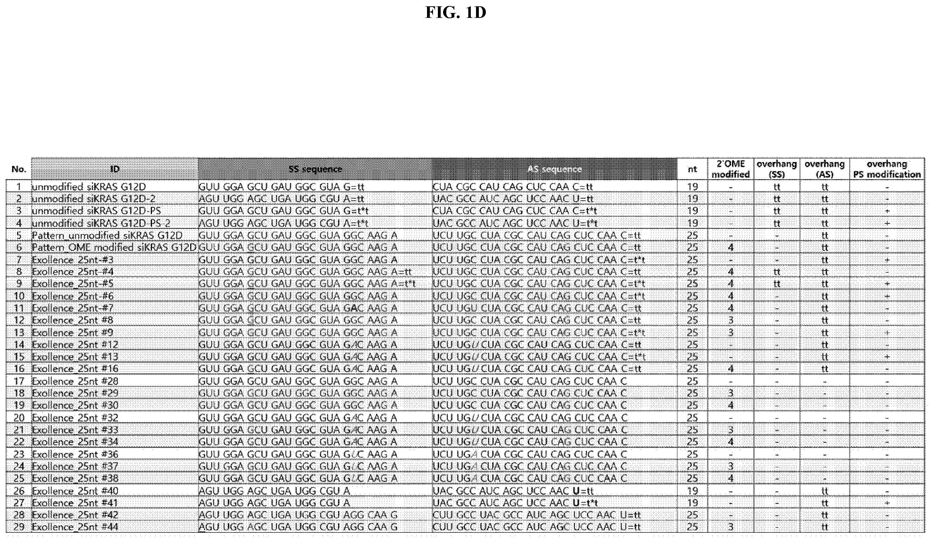

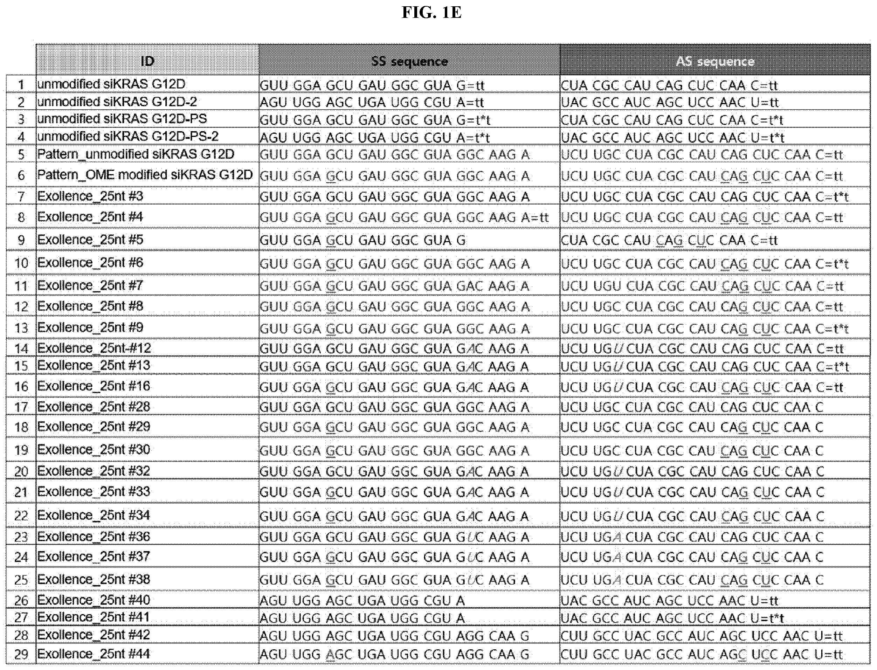

The results are shown as in FIGS. 1A to 1C, and thus 29 types of siRNAs are identified as shown in Tables 3 and 4 (FIGS. 1D and 1E) (Here, PS modification means that G/C is changed to *A/U*, represented in italics (A: blue/U: green). In addition, 2' OMe modification is underlined (in red)).

[Table 3]

| No. | ID | SS sequence |
|---|---|---|
| 1 | unmodified siKRAS G12D | GUU GGA GCU GAU GGC GUA G=tt |
| 2 | unmodified siKRAS G12D-2 | AGU UGG AGC UGA UGG CGU A=tt |
| 3 | unmodified siKRAS G12D-PS | GUU GGA GCU GAU GGC GUA G=t*t |
| 4 | unmodified siKRAS G12D-PS-2 | AGU UGG AGC UGA UGG CGU A=t*t |
| 5 | Pattern_unmodified siKRAS G12D | GUU GGA GCU GAU GGC GUA GGC AAG A |
| 6 | Pattern_OME modified siKRAS G12D | GUU GGA GCU GAU GGC GUA GGC AAG A |
| 7 | Exollence_25nt #3 | GUU GGA GCU GAU GGC GUA GGC AAG A |
| 8 | Exollence_25nt #4 | GUU GGA GCU GAU GGC GUA GGC AAG A=tt |
| 9 | Exollence_25nt #5 | GUU GGA GCU GAU GGC GUA GGC AAG A=t*t |
| 10 | Exollence_25nt #6 | GUU GGA GCU GAU GGC GUA GGC AAG A |
| 11 | Exollence_25nt #7 | GUU GGA GCU GAU GGC GUA GAC AAG A |

(continued)

| No. | ID | SS sequence |
|---|---|---|
| 12 | Exollence_25nt #8 | GUU GGA GCU GAU GGC GUA GGC AAG A |
| 13 | Exollence_25nt #9 | GUU GGA GCU GAU GGC GUA GGC AAG A |
| 14 | Exollence_25nt-#12 | GUU GGA GCU GAU GGC GUA GAC AAG A |
| 15 | Exollence_25nt #13 | GUU GGA GCU GAU GGC GUA GAC AAG A |
| 16 | Exollence_25nt #16 | GUU GGA GCU GAU GGC GUA GAC AAG A |
| 17 | Exollence_25nt #28 | GUU GGA GCU GAU GGC GUA GGC AAG A |
| 18 | Exollence_25nt #29 | GUU GGA GCU GAU GGC GUA GGC AAG A |
| 19 | Exollence_25nt #30 | GUU GGA GCU GAU GGC GUA GGC AAG A |
| 20 | Exollence_25nt #32 | GUU GGA GCU GAU GGC GUA GAC AAG A |
| 21 | Exollence_25nt #33 | GUU GGA GCU GAU GGC GUA GAC AAG A |
| 22 | Exollence_25nt #34 | GUU GGA GCU GAU GGC GUA GAC AAG A |
| 23 | Exollence_25nt #36 | GUU GGA GCU GAU GGC GUA GUC AAG A |
| 24 | Exollence_25nt #37 | GUU GGA GCU GAU GGC GUA GUC AAG A |
| 25 | Exollence_25nt #38 | GUU GGA GCU GAU GGC GUA GUC AAG A |
| 26 | Exollence_25nt #40 | AGU UGG AGC UGA UGG CGU A |
| 27 | Exollence_25nt #41 | AGU UGG AGC UGA UGG CGU A |
| 28 | Exollence_25nt #42 | AGU UGG AGC UGA UGG CGU AGG CAA G |
| 29 | Exollence_25nt #44 | AGU UGG AGC UGA UGG CGU AGG CAA G |

[Table 4]

| No. | ID | AS sequence |
|---|---|---|
| 30 | unmodified siKRAS G12D | CUA CGC CAU CAG CUC CAA C=tt |
| 31 | unmodified siKRAS G12D-2 | UAC GCC AUC AGC UCC AAC U=tt |
| 32 | unmodified siKRAS G12D-PS | CUA CGC CAU CAG CUC CAA C=t*t |
| 33 | unmodified siKRAS G12D-PS-2 | UAC GCC AUC AGC UCC AAC U=t*t |
| 34 | Pattern_unmodified siKRAS G12D | UCU UGC CUA CGC CAU CAG CUC CAA C=tt |
| 35 | Pattern_OME modified siKRAS G12D | UCU UGC CUA CGC CAU CAG CUC CAA C=tt |
| 36 | Exollence_25nt #3 | UCU UGC CUA CGC CAU CAG CUC CAA C=t*t |
| 37 | Exollence_25nt #4 | UCU UGC CUA CGC CAU CAG CUC CAA C=tt |
| 38 | Exollence_25nt #5 | UCU UGC CUA CGC CAU CAG CUC CAA C=t*t |
| 39 | Exollence_25nt #6 | UCU UGC CUA CGC CAU CAG CUC CAA C=t*t |
| 40 | Exollence_25nt #7 | UCU UGU CUA CGC CAU CAG CUC CAA C=tt |
| 41 | Exollence_25nt #8 | UCU UGC CUA CGC CAU CAG CUC CAA C=tt |

(continued)

| No. | ID | AS sequence |
|---|---|---|
| 42 | Exollence_25nt #9 | UCU UGC CUA CGC CAU CA<u>G</u> C<u>U</u>C CAA C=t*t |
| 43 | Exollence_25nt-#12 | UCU UGU CUA CGC CAU CAG CUC CAA C=tt |
| 44 | Exollence_25nt #13 | UCU UG*U* CUA CGC CAU CAG CUC CAA C=t*t |
| 45 | Exollence_25nt #16 | UCU UGU CUA CGC CAU <u>CAG</u> C<u>U</u>C CAA C=tt |
| 46 | Exollence_25nt #28 | UCU UGC CUA CGC CAU CAG CUC CAA C |
| 47 | Exollence_25nt #29 | UCU UGC CUA CGC CAU CA<u>G</u> C<u>U</u>C CAA C |
| 48 | Exollence_25nt #30 | UCU UGC CUA CGC CAU <u>CAG</u> C<u>U</u>C CAA C |
| 49 | Exollence_25nt #32 | UCU UG*U* CUA CGC CAU CAG CUC CAA C |
| 50 | Exollence_25nt #33 | UCU UG*U* CUA CGC CAU CA<u>G</u> C<u>U</u>C CAA C |
| 51 | Exollence_25nt #34 | UCU UG*U* CUA CGC CAU <u>CAG</u> C<u>U</u>C CAA C |
| 52 | Exollence_25nt #36 | UCU UG*A* CUA CGC CAU CAG CUC CAA C |
| 53 | Exollence_25nt #37 | UCU UG*A* CUA CGC CAU CA<u>G</u> C<u>U</u>C CAA C |
| 54 | Exollence_25nt #38 | UCU UG*A* CUA CGC CAU <u>CAG</u> C<u>U</u>C CAA C |
| 55 | Exollence_25nt #40 | UAC GCC AUC AGC UCC AAC U=tt |
| 56 | Exollence_25nt #41 | UAC GCC AUC AGC UCC AAC U=t*t |
| 57 | Exollence_25nt #42 | CUU GCC UAC GCC AUC AGC UCC AAC U=tt |
| 58 | Exollence_25nt #44 | CUU GCC UAC GCC AUC AG<u>C</u> U<u>C</u>C AAC U=tt |

Example 1-3. Identification of three groups of lead substances with common pattern

It was analyzed whether the lead substances identified in Examples 1-2 had a common pattern. As a result, it was confirmed that the lead substances are classified into three groups (hereinafter, Groups 1 to 3) (Tables 5 to 7).

[Table 5]

| | ID | SS sequence | AS sequence | nt | 2'OME modified | overhang (SS) | overhang (AS) | overhang PS modification |
|---|---|---|---|---|---|---|---|---|
| 1 | Exollence_25nt #41 | <u>A</u>GU UGG AGC UGA UGG CGU A | UAC GCC AUC AGC UCC AAC <u>U</u>=t*t | 19 | 0 | - | tt | + |
| 4 | Exollence_25nt-#3 | GUU GGA GCU GAU GGC GUA GGC AAG A | UCU UGC CUA CGC CAU CAG CUC CAA C=t*t | 25 | 0 | - | tt | + |
| 6 | Exollence_25nt #28 | GUU GGA GCU GAU GGC GUA GGC AAG A | UCU UGC CUA CGC CAU CAG CUC CAA C | 25 | 0 | - | - | - |
| 9 | Exollence_25nt-#13 | GUU GGA GCU GAU GGC GUA G*A*C AAG A | UCU UG*U* CUA CGC CAU CAG CUC CAA C=t*t | 25 | 0 | - | tt | + |
| 13 | unmodified siKRAS G12D-PS-2 | <u>A</u>GU UGG AGC UGA UGG CGU A=t*t | UAC GCC AUC AGC UCC AAC <u>U</u>=t*t | 19 | 0 | tt | tt | + |
| 14 | Exollence_25nt #40 | <u>A</u>GU UGG AGC UGA UGG CGU A | UAC GCC AUC AGC UCC AAC <u>U</u>=tt | 19 | 0 | - | tt | - |
| 15 | unmodified siKRAS G12D-2 | <u>A</u>GU UGG AGC UGA UGG CGU A=tt | UAC GCC AUC AGC UCC AAC <u>U</u>=tt | 19 | 0 | tt | tt | - |
| 16 | Exollence_25nt-#12 | GUU GGA GCU GAU GGC GUA G*A*C AAG A | UCU UG*U* CUA CGC CAU CAG CUC CAA C=tt | 25 | 0 | - | tt | - |
| 17 | Exollence_25nt #32 | GUU GGA GCU GAU GGC GUA G*A*C AAG A | UCU UG*U* CUA CGC CAU CAG CUC CAA C | 25 | 0 | - | - | - |
| 21 | Exollence_25nt #36 | GUU GGA GCU GAU GGC GUA G*U*C AAG A | UCU UG*A* CUA CGC CAU CAG CUC CAA C | 25 | 0 | - | - | - |
| 22 | unmodified siKRAS G12D-PS | GUU GGA GCU GAU GGC GUA G=t*t | CUA CGC CAU CAG CUC CAA C=t*t | 19 | 0 | tt | tt | + |
| 23 | Exollence_25nt #42 | <u>A</u>GU UGG AGC UGA UGG CGU AGG CAA G | CUU GCC UAC GCC AUC AGC UCC AAC U=tt | 25 | 0 | - | tt | - |
| 26 | unmodified siKRAS G12D | GUU GGA GCU GAU GGC GUA G=tt | CUA CGC CAU CAG CUC CAA C=tt | 19 | 0 | tt | tt | - |
| 29 | Pattern_unmodified siKRAS G12D | GUU GGA GCU GAU GGC GUA GGC AAG A | UCU UGC CUA CGC CAU CAG CUC CAA C=tt | 25 | 0 | - | tt | - |

[Table 6]

| | ID | SS sequence | AS sequence | nt | 2'OME modified | overhang (SS) | overhang (AS) | overhang PS modification |
|---|---|---|---|---|---|---|---|---|
| 2 | Exollence_25nt-#8 | GUU GGA GCU GAU GGC GUA GGC AAG A | UCU UGC CUA CGC CAU CAG CUC CAA C=tt | 25 | 3 | - | tt | - |
| 3 | Exollence_25nt-#9 | GUU GGA GCU GAU GGC GUA GGC AAG A | UCU UGC CUA CGC CAU CAG CUC CAA C=t*t | 25 | 3 | - | tt | + |
| 8 | Exollence_25nt #29 | GUU GGA GCU GAU GGC GUA GGC AAG A | UCU UGC CUA CGC CAU CAG CUC CAA C | 25 | 3 | - | - | - |
| 12 | Exollence_25nt #33 | GUU GGA GCU GAU GGC GUA GAC AAG A | UCU UGU CUA CGC CAU CAG CUC CAA C | 25 | 3 | - | - | - |
| 24 | Exollence_25nt #37 | GUU GGA GCU GAU GGC GUA GUC AAG A | UCU UGA CUA CGC CAU CAG CUC CAA C | 25 | 3 | - | - | - |
| 30 | Exollence_25nt #44 | AGU UGG AGC UGA UGG CGU AGG CAA G | CUU GCC UAC GCC AUC AGC UCC AAC U=tt | 25 | 3 | - | tt | - |

[Table 7]

| | ID | SS sequence | AS sequence | nt | 2'OME modified | overhang (SS) | overhang (AS) | overhang PS modification |
|---|---|---|---|---|---|---|---|---|
| 5 | Exollence_25nt-#6 | GUU GGA GCU GAU GGC GUA GGC AAG A | UCU UGC CUA CGC CAU CAG CUC CAA C=t*t | 25 | 4 | - | tt | + |
| 7 | Exollence_25nt #30 | GUU GGA GCU GAU GGC GUA GGC AAG A | UCU UGC CUA CGC CAU CAG CUC CAA C | 25 | 4 | - | - | - |
| 10 | Exollence_25nt-#7 | GUU GGA GCU GAU GGC GUA GAC AAG A | UCU UGU CUA CGC CAU CAG CUC CAA C=tt | 25 | 4 | - | tt | - |
| 11 | Exollence_25nt-#16 | GUU GGA GCU GAU GGC GUA GAC AAG A | UCU UGU CUA CGC CAU CAG CUC CAA C=tt | 25 | 4 | - | tt | - |
| 18 | Exollence_25nt #34 | GUU GGA GCU GAU GGC GUA GAC AAG A | UCU UGU CUA CGC CAU CAG CUC CAA C | 25 | 4 | - | - | - |
| 19 | Pattern_OME modified siKRAS G12D | GUU GGA GCU GAU GGC GUA GGC AAG A | UCU UGC CUA CGC CAU CAG CUC CAA C=tt | 25 | 4 | - | tt | - |
| 20 | Exollence_25nt-#4 | GUU GGA GCU GAU GGC GUA GGC AAG A=tt | UCU UGC CUA CGC CAU CAG CUC CAA C=tt | 25 | 4 | tt | tt | - |
| 27 | Exollence_25nt-#5 | GUU GGA GCU GAU GGC GUA GGC AAG A=t*t | UCU UGC CUA CGC CAU CAG CUC CAA C=t*t | 25 | 4 | tt | tt | + |
| 28 | Exollence_25nt #38 | GUU GGA GCU GAU GGC GUA GUC AAG A | UCU UGA CUA CGC CAU CAG CUC CAA C | 25 | 4 | - | - | - |

According to the above results, it was confirmed that, for Group 1, the sense strand is 19 to 25 nt long, and the anti-sense strand is 21 to 27 nt long.

In addition, for Group 2, the sense strand is 19 to 25 nt long, and the anti-sense strand is 21 to 27 nt long. The sense strand did not have a tt overhang, and chemical modification was made by the 2'-OMe method. The 2'-OMe-chemically-modified nucleotide at the 7th position from the 5' end of the sense strand (the nucleotide in the anti-sense strand binding to this position was not modified) was observed, and it was confirmed that the nucleotide at the 9th position from the 5' end of the sense strand was not modified. In addition, it was confirmed that the 18th and 20th nucleotides from the 5' end of the anti-sense strand were chemically modified with 2'-OMe.

Finally, for Group 3, the sense strand is 19 to 25 nt long, and the anti-sense strand is 21 to 27 nt long. Chemical modification was made by the 2'-OMe method. The 2'-OMe-chemically-modified nucleotide at the 7th position from the 5' end of the sense strand (the nucleotide of the anti-sense strand binding to this position was not modified) was observed, and it was confirmed that the nucleotide at the 9th position from the 5' end of the sense strand was not modified. In addition, it was confirmed that the 16th, 18th, and 20th nucleotides from the 5' end of the anti-sense strand were chemically modified with 2'-OMe.

**Example 2. Confirmation of anticancer effects of 29 types of siRNAs on pancreatic cancer cell lines**

Anticancer effects exhibited when a pancreatic cancer cell line was treated with the 29 types of siRNAs finally confirmed in Example 1 were confirmed. Specifically, the pancreatic cancer cell line was cultured in a 100 cm$^2$ dish using DMEM (10% FBS, 1% penicillin-streptomycin). In addition, a colorectal cancer cell line was cultured using RPMI medium (10% FBS, 1% penicillin-streptomycin). Both pancreatic cancer and colorectal cancer cell lines were cultured under conditions of 37 °C and 5% $CO_2$. When cells reached a confluence of 70 to 80%, the cells were washed with PBS and suspended with trypsin-EDTA. The suspension of the cells was then stopped using a culture medium, and the cell suspension was centrifuged (1,000 rpm, 5 min) to collect the cells. The cells collected by centrifugation were resuspended with a fresh culture medium, and to confirm a cell survival rate, a cell count was measured, and then the cells were inoculated at a concentration of 10,000 cells/well in a 96-well plate. The cells were incubated in 5% $CO_2$ at 37 °C for 24 hours and attached.

For siRNAs to be tested and the control siRNA, 75 μL of Opti-MEM medium and each siRNA (prepared to have a final concentration of 10 nM) were mixed. 75 μL of Opti-MEM medium and 2.5 μL of Lipofectamine RNAiMAX were mixed in a new tube. A siRNA-Lipofectamine complex was formed by combining and reacting the two mixtures at room temperature for 20 minutes, and the siRNA-Lipofectamine complex was added to each well. The cells were incubated in 5% $CO_2$ at 37 °C.

10 μL of WST-8 reagent was added to each well every 48 hours, and the plate was incubated for 1 hour in 5% $CO_2$ at 37 °C. Using a microplate reader, the absorbance of each well was measured at 450 nm and the background absorbance was measured at 650 nm for correction. The absorbance value of the treated sample was compared with that of the control, and a relative cell growth rate was calculated by the following equation.

$$\text{Equation: (absorbance of treated sample/absorbance of control)} \times 100$$

As a result, the apoptotic effects of Group 1 on the pancreatic cancer cell lines AsPC-1, HPAFII, and PANC-1 were confirmed as shown in FIGS. 2A to 2C. The results of the same experiment for Group 2 are shown in FIG. 2D. Finally, the experimental results of Group 3 are shown in FIGS. 2E to 2G. It was confirmed that all of Groups 1 to 3 reduced pancreatic cancer cell growth in groups treated with different lead substances compared to a control treated with scrambled siRNA (Bioneer).

[Table 8]

| No. | Name | Sequence |
|---|---|---|
| 61 | siScramble siRNA_SS | GAG GUG UAG GGC UGU ACG U=tt |
| 62 | siScramble siRNA_AS | ACG UAG AGC CCU AGA CCU C=tt |

**Example 3. Confirmation of anticancer effects of 29 types of siRNAs on colorectal cancer cell lines**

The anticancer effects of 29 types of siRNAs on colorectal cancer were analyzed by applying the same experimental method used in Example 2.

As a result, the apoptotic effects of Group 1 on colorectal cancer cell lines SNU-C2B, LS174T, and LS513 were confirmed as shown in FIGS. 3A to 3C. The results of the same experiment for Group 2 are shown in FIG. 3D. Finally, the experimental results for Group 3 are shown in FIGS. 3E to 3G. It was confirmed that all Groups 1 to 3 reduced colorectal cancer cell growth in groups treated with different lead substances compared to a control treated with scrambled siRNA.

According to Examples 2 and 3, the 29 lead substances of the present invention inhibited cancer cell growth rates, confirming that they exhibit anticancer effects on colorectal cancer and pancreatic cancer.

**Example 4. Confirmation of KRAS mRNA expression levels for pancreatic cancer cell lines**

Effects on KRAS mRNA expression when a pancreatic cancer cell line was treated with each siRNA of the present invention were confirmed. Specifically, to confirm the change in intracellular KRAS mRNA expression due to siRNA treatment, a cell count was measured, the cells were inoculated at 100,000 cells/well in a 12-well plate. The cells were incubated in 5% $CO_2$ at 37 °C for 24 hours and attached.

As siRNAs to be tested, unmodified siKRAS G12D, unmodified siKRAS G12D-2, unmodified siKRAS G12D-PS (1 to 3 in Table 3), and Pattern_OMe modified siKRAS G12D (6 in Table 3) were used. In addition, as a control, Alnylam_unmodified siKRAS G12D was used. Here, Alnylam_unmodified siKRAS G12D is siRNA that was identified from the 105 types of siRNAs of the present invention, but not selected as one of the final 29 lead substances. Alnylam_unmodified siKRAS G12D has the following characteristics: 21 nt, no 2'OMe modification, no overhang(SS), overhang(AS) tt, and overhang PS modification (-).

[Table 9]

| No. | Name | Sequence |
|---|---|---|
| 63 | Alnylam_unmodified siKRAS G12D_SS | GUU GGA GCU GAU GGC GUA GGC |
| 64 | Alnylam_unmodified siKRAS G12D_AS | GCC UAC GCC AUC AGC UCC AAC=tt |

For the experimental group siRNA and the control siRNA, 75 μL of Opti-MEM medium and each siRNA (prepared to have a final concentration of 10 nM) were mixed. 75 μL of Opti-MEM medium and 2.5 μL of Lipofectamine RNAiMAX

were mixed in a new tube. A siRNA-Lipofectamine complex was formed by combining and reacting the two mixtures at room temperature for 20 minutes. The siRNA-Lipofectamine complex was added to each well and the cells were incubated in 5% $CO_2$ at 37 °C.

After 24-hour incubation, the existing culture medium was removed and 1000 $\mu$L of QIAzol Lysis Reagent was added to each well to lyse the cells. A solution obtained by completely homogenizing the cells by pipetting was transferred to a 1.5 mL tube and left at room temperature for 5 minutes to induce complete lysis. 200 $\mu$L of chloroform was added and mixed vigorously for 15 seconds, the mixture was left at room temperature for 2 to 3 minutes and centrifuged at 12,000 x g and 4 °C for 15 minutes, and then a supernatant (aqueous layer) was carefully transferred to a new tube. 2-propanol was added to the supernatant and mixed to precipitate RNA, followed by centrifugation at 12,000 x g for 15 minutes at 4 °C. The supernatant was removed, 70% ethanol was added, RNA was washed, centrifugation was performed at 12,000 x g for 10 minutes at 4 °C, and the supernatant was completely removed. The RNA pellet was dissolved in nuclease-free water, and the concentration and purity of extracted RNA were measured using Nanodrop.

cDNA was synthesized by a cDNA synthesis kit using the extracted RNA (1 $\mu$g). Real time PCR was performed using primers for a designed target gene and a housekeeping gene (GAPDH was used as a housekeeping gene). PCR was performed under the following conditions using a real time PCR instrument: initial denaturation: 95 °C, 2 min, 40 cycles: 95 °C 15 sec, 60 °C 1 min. To analyze the results, the relative expression level of the target gene was calculated using the $\Delta\Delta Ct$ value.

As a result, it was seen that the KRAS mRNA expression levels of unmodified siKRAS G12D, unmodified siKRAS G12D-2, unmodified siKRAS G12D-PS (1 to 3 in Table 3), and Pattern_OMe modified siKRAS G12D (6 in Table 3) were significantly reduced (FIG. 4A). On the other hand, it was shown that the control Alnylam_unmodified siKRAS G12D does not have a significant effect on KRAS mRNA expression (FIG. 4B). (Here, siRNA 0 nM indicates a control, and siScramble indicates siRNA 10 nM.)

## Example 5. Confirmation of tumor size inhibitory effect in pancreatic cancer animal models

A tumor size inhibitory effect when a pancreatic cancer animal model was treated with each siRNA of the present invention was confirmed. Specifically, $1\times10^7$ KPC-luc cells were prepared in 100 $\mu$L PBS, 6 to 8-week-old female nude mice were anesthetized, and $1\times10^7/100$ $\mu$L of the cells were injected subcutaneously between the skin and muscle layers of the anesthetized mice using a 31-gauge syringe. After injection, the mice were allowed to recover in a recovery room. After cell injection, the length (L) and width (W) of a tumor were measured using digital calipers. A tumor volume was calculated using the following equation: tumor volume, $(mm^3)=L\times W^2\times0.5$.

When the tumor volume reached 100 $mm^3$, the injection of a therapeutic agent began, and the mice with the tumor size of 100 $mm^3$ were randomly divided into two groups, a treatment group and a control. The treatment group was injected with the therapeutic agent via the tail vein three times (Monday, Wednesday and Friday) a week. Here, the treatment group used Exollence_25nt #6 (10 in Table 3) as an experimental group, and Exollence_25nt #1 (Table 10), siScramble (negative control), and Ctl (control) were used as comparison groups. Here, Exollence_25nt #1 is a material that did not exhibit the apoptotic effects of KRAS G12D in the experiment of measuring a cell survival rate by treating cells, and was used as a negative control. Exollence_25nt #1 has the following characteristics: 25 nt, no 2'OMe modification, overhang(SS) tt, overhang(AS) tt, and overhang PS modification (-).

[Table 10]

| No. | Name | Sequence |
|-----|------|----------|
| 65 | Exollence_25nt #1_SS | GUU GGA GCU GAU GGC GUA GGC AAG A=tt |
| 66 | Exollence_25nt #1_AS | UCU UGC CUA CGC CAU CAG CUC CAA C=tt |

siRNA was injected at an injection dose of 20 $\mu$g/100 $\mu$L, and the same amount of sterile PBS was injected into the control at the same cycle. When injecting the therapeutic agent, the mouse was placed on a warming pad, the tail was warmed to dilate the tail vein, and the therapeutic agent was injected into the tail vein using a 29-gauge needle.

During the injection period, the tumor length and width were measured twice a week, and the tumor volume was calculated. To confirm side effects, the body weight and general health conditions of a mouse were regularly monitored. After the last injection of the therapeutic agent, the tumor was removed and weighed.

As a result, it was shown that Exollence_25nt #1, siScramble, and the control did not reduce tumor sizes at all in the pancreatic cancer cell line (KPC-luc) xenograft mouse model. However, when Exollence_25nt #6 (10 in Table 3) was treated, it was shown that the tumor size and weight were reduced approximately 60% (FIG. 5A). At the same time, when Exollence_25nt #6 (10 in Table 3) was treated, no change in body weight was observed, similar to other comparison groups including the control (FIG. 5B), the above results confirm the anticancer effects of the lead substance of the present invention in animal experiments.

The aforementioned description of the present invention is provided by way of example and those skilled in the art will understand that the present invention can be easily changed or modified into other specified forms without change or modification of the technical spirit or essential characteristics of the present invention. Therefore, it should be understood that the aforementioned examples are only provided by way of example and not provided to limit the present invention.

[Industrial Applicability]

The present invention relates to a pharmaceutical composition for preventing or treating cancer comprising siRNA as an active ingredient, which was found to significantly inhibit the cell proliferation effect of colorectal cancer or pancreatic cancer, thereby exhibiting excellent anticancer effects and reducing the expression level of KRAS mRNA. Additionally, since it reduces the size and weight of tumors while maintaining the body weight of the subject, it may be usefully employed as an excellent anticancer therapeutic agent, and thus industrial applicability is acknowledged.

**Claims**

1. A pharmaceutical composition for preventing or treating cancer, comprising one or more selected from the group consisting of siRNAs having any one nucleotide sequence selected from the group consisting of SEQ ID NOs: 1 to 29.

2. The pharmaceutical composition of claim 1, wherein the group consisting of siRNAs below have a sense strand that is 19 to 25 nt long and an anti-sense strand that is 21 to 27 nt long:

   1) siRNA having any one nucleotide sequence selected from the group consisting of SEQ ID NOs: 1 to 5;
   2) siRNA having a nucleotide sequence represented by SEQ ID NO: 7;
   3) siRNA having any one nucleotide sequence selected from the group consisting of SEQ ID NOs: 14, 15, and 17; and
   4) siRNA having any one nucleotide sequence selected from the group consisting of SEQ ID NOs: 20, 23, and 26 to 28.

3. The pharmaceutical composition of claim 1, wherein the group consisting of siRNA having a nucleotide sequence represented by SEQ ID NO: 12, siRNA having a nucleotide sequence represented by SEQ ID NO: 13, siRNA having a nucleotide sequence represented by SEQ ID NO: 18, siRNA having a nucleotide sequence represented by SEQ ID NO: 21, siRNA having a nucleotide sequence represented by SEQ ID NO: 24, and siRNA having a nucleotide sequence represented by SEQ ID NO: 29 has the following characteristics:

   a) having a sense strand that is 19 to 25 nt long and an anti-sense strand that is 21 to 27 nt long;
   b) having no tt overhang in a sense strand;
   c) having a 2'-OMe-chemically modified nucleotide at the 7th position from the 5' end of a sense strand, and a nucleotide in an anti-sense strand binding to this position, which is not chemically modified with 2'-OMe;
   d) having a nucleotide at the 9th position from the 5' end of the sense strand, which is not chemically modified with 2'-OMe; and
   e) having 2'-OMe-chemically modified nucleotides at the 18th and 20th positions from the 5' end of the anti-sense strand.

4. The pharmaceutical composition of claim 1, wherein the group consisting of siRNA comprising the nucleotide sequence represented by SEQ ID NO: 6, siRNA comprising the nucleotide sequence represented by SEQ ID NO: 8, siRNA comprising the nucleotide sequence represented by SEQ ID NO: 9, siRNA comprising the nucleotide sequence represented by SEQ ID NO: 10, siRNA comprising the nucleotide sequence represented by SEQ ID NO: 11, siRNA comprising the nucleotide sequence represented by SEQ ID NO: 16, siRNA comprising the nucleotide sequence represented by SEQ ID NO: 19, siRNA comprising the nucleotide sequence represented by SEQ ID NO: 22, and siRNA comprising the nucleotide sequence represented by SEQ ID NO: 25 has the following characteristics:

   a) having a sense strand that is 19 to 25 nt long and an anti-sense strand that is 21 to 27 nt long;
   b) having a 2'-OMe-chemically modified nucleotide at the 7th position from the 5' end of a sense strand, and a nucleotide in an anti-sense strand binding to this position, which is not chemically modified with 2'-OMe;
   c) having a nucleotide at the 9th position from the 5' end of the sense strand, which is not chemically modified with 2'-OMe; and

d) having 2'-OMe-chemically modified nucleotides at the 16th, 18th and 20th positions from the 5' end of the anti-sense strand.

5. The pharmaceutical composition of claim 1, wherein the cancer is one selected from the group consisting of pancreatic cancer, colorectal cancer, squamous cell carcinoma, lung cancer, adenocarcinoma of the lung, peritoneal cancer, skin cancer, skin or intraocular melanoma, rectal cancer, anal cancer, esophageal cancer, small intestinal cancer, endocrine cancer, parathyroid cancer, adrenal cancer, soft tissue sarcoma, urethral cancer, blood cancer, liver cancer, gastric cancer, glioblastoma, cervical cancer, ovarian cancer, bladder cancer, liver tumors, breast cancer, colon cancer, endometrial cancer, uterine cancer, salivary gland cancer, kidney cancer, prostate cancer, vulvar cancer, thyroid cancer, head and neck cancer, and brain cancer.

6. The pharmaceutical composition of claim 1, wherein the composition inhibits the proliferation of cancer cells.

7. The pharmaceutical composition of claim 1, wherein the composition reduces tumor size and weight.

8. The pharmaceutical composition of claim 1, wherein the composition inhibits KRAS mRNA expression.

9. A kit for preventing or treating cancer, comprising a composition comprising one or more selected from the group consisting of siRNAs having any one nucleotide sequence selected from the group consisting of SEQ ID NOs: 1 to 29 as an active ingredient, and an instruction manual.

10. A method of treating cancer, comprising:
administering a pharmaceutically effective amount of a composition comprising one or more selected from the group consisting of siRNAs having any one nucleotide sequence selected from the group consisting of SEQ ID NOs: 1 to 29 as an active ingredient to a subject in need thereof.

11. A use of a composition comprising one or more selected from the group consisting of siRNAs having any one nucleotide sequence selected from the group consisting of SEQ ID NOs: 1 to 29 as an active ingredient for preventing or treating cancer.

12. A use of a composition comprising one or more selected from the group consisting of siRNAs having any one nucleotide sequence selected from the group consisting of SEQ ID NOs: 1 to 29 as an active ingredient for preparing a formulation for preventing or treating cancer.

FIG. 1A

**FIG. 1B**

siScramble (Ctl)
Exollence_25nt-#9
Exollence_25nt-#3
Exollence_25nt #28
Exollence_25nt #29
Exollence_25nt-#7
Exollence_25nt-#16
Exollence_25nt #32
Exollence_25nt-#12
Exollence_25nt-#80
Exollence_25nt #36
Exollence_25nt-#79
Exollence_25nt-#4
Exollence_25nt-#77
Exollence_25nt #42
Exollence_25nt-#63
25nt #70
Exollence_25nt #31
Exollence_25nt-#5
Pattern_unmodified siKRAS..
Exollence_25nt #44
Exollence_25nt #50
Exollence_25nt #54
25nt #82
Exollence_19nt-#2
Exollence_25nt #35
Exollence_25nt #43
25nt #69
Exollence_19nt-#4
Exollence_25nt #53
Exollence_25nt-#11
Exollence_25nt-#20
Exollence_25nt-#17
Exollence_25nt-#14
Exollence_25nt #47
Exollence_25nt #52
Exollence_25nt #58
Exollence_19nt-#5
Exollence_25nt-#22
Exollence_25nt-#25
25nt #72
Exollence_19nt-#1
Exollence_25nt #46
Exollence_25nt-#23
Exollence_25nt #60
Exollence_25nt #51
Exollence_25nt #59
Exollence_25nt-#27
Exollence_19nt-#3
Exollence_25nt #57
Exollence_19nt-#7
modified siKRAS G12D-PS
Exollence_19nt-#8

0.0 10.0 20.0 30.0 40.0 50.0 60.0 70.0 80.0 90.0 100.0 110.0 120.0 130.0

FIG. 1C

**FIG. 1D**

| No. | ID | SS sequence | AS sequence | nt | 2'OME modified | overhang (SS) | overhang (AS) | overhang PS modification |
|---|---|---|---|---|---|---|---|---|
| 1 | unmodified siKRAS G12D | GUU GGA GCU GAU GGC GUA G=tt | CUA CGC CAU CAG CUC CAA C=tt | 19 | - | tt | tt | - |
| 2 | unmodified siKRAS G12D-2 | AGU UGG AGC UGA UGG CGU A=tt | UAC GCC AUC AGC UCC AAC U=tt | 19 | - | tt | tt | - |
| 3 | unmodified siKRAS G12D-PS | GUU GGA GCU GAU GGC GUA G=t*t | CUA CGC CAU CAG CUC CAA C=t*t | 19 | - | tt | tt | + |
| 4 | unmodified siKRAS G12D-PS-2 | AGU UGG AGC UGA UGG CGU A=t*t | UAC GCC AUC AGC UCC AAC U=t*t | 19 | - | tt | tt | + |
| 5 | Pattern_unmodified siKRAS G12D | GUU GGA GCU GAU GGC GUA GGC AAG A | UCU UGC CUA CGC CAU CAG CUC CAA C=tt | 25 | - | - | tt | - |
| 6 | Pattern_OME modified siKRAS G12D | GUU GGA GCU GAU GGC GUA GGC AAG A | UCU UGC CUA CGC CAU CAG CUC CAA C=tt | 25 | 4 | - | tt | - |
| 7 | Exollence_25nt-#3 | GUU GGA GCU GAU GGC GUA GGC AAG A | UCU UGC CUA CGC CAU CAG CUC CAA C=t*t | 25 | - | - | tt | + |
| 8 | Exollence_25nt-#4 | GUU GGA GCU GAU GGC GUA GGC AAG A=tt | UCU UGC CUA CGC CAU CAG CUC CAA C=tt | 25 | 4 | tt | tt | - |
| 9 | Exollence_25nt-#5 | GUU GGA GCU GAU GGC GUA GGC AAG A=t*t | UCU UGC CUA CGC CAU CAG CUC CAA C=t*t | 25 | 4 | tt | tt | + |
| 10 | Exollence_25nt-#6 | GUU GGA GCU GAU GGC GUA GGC AAG A | UCU UGC CUA CGC CAU CAG CUC CAA C=t*t | 25 | 4 | - | tt | + |
| 11 | Exollence_25nt-#7 | GUU GGA GCU GAU GGC GUA GAC AAG A | UCU UGU CUA CGC CAU CAG CUC CAA C=tt | 25 | 4 | - | tt | - |
| 12 | Exollence_25nt-#8 | GUU GGA GCU GAU GGC GUA GGC AAG A | UCU UGC CUA CGC CAU CAG CUC CAA C=tt | 25 | 3 | - | tt | - |
| 13 | Exollence_25nt-#9 | GUU GGA GCU GAU GGC GUA GGC AAG A | UCU UGC CUA CGC CAU CAG CUC CAA C=t*t | 25 | 3 | - | tt | + |
| 14 | Exollence_25nt #12 | GUU GGA GCU GAU GGC GUA GAC AAG A | UCU UGU CUA CGC CAU CAG CUC CAA C=tt | 25 | - | - | tt | - |
| 15 | Exollence_25nt #13 | GUU GGA GCU GAU GGC GUA GAC AAG A | UCU UGU CUA CGC CAU CAG CUC CAA C=t*t | 25 | - | - | tt | + |
| 16 | Exollence_25nt #16 | GUU GGA GCU GAU GGC GUA GAC AAG A | UCU UGU CUA CGC CAU CAG CUC CAA C=tt | 25 | 4 | - | tt | - |
| 17 | Exollence_25nt #28 | GUU GGA GCU GAU GGC GUA GGC AAG A | UCU UGC CUA CGC CAU CAG CUC CAA C | 25 | - | - | - | - |
| 18 | Exollence_25nt #29 | GUU GGA GCU GAU GGC GUA GGC AAG A | UCU UGC CUA CGC CAU CAG CUC CAA C | 25 | 3 | - | - | - |
| 19 | Exollence_25nt #30 | GUU GGA GCU GAU GGC GUA GGC AAG A | UCU UGC CUA CGC CAU CAG CUC CAA C | 25 | 4 | - | - | - |
| 20 | Exollence_25nt #32 | GUU GGA GCU GAU GGC GUA GAC AAG A | UCU UGU CUA CGC CAU CAG CUC CAA C | 25 | - | - | - | - |
| 21 | Exollence_25nt #33 | GUU GGA GCU GAU GGC GUA GAC AAG A | UCU UGU CUA CGC CAU CAG CUC CAA C | 25 | 3 | - | - | - |
| 22 | Exollence_25nt #34 | GUU GGA GCU GAU GGC GUA GAC AAG A | UCU UGU CUA CGC CAU CAG CUC CAA C | 25 | 4 | - | - | - |
| 23 | Exollence_25nt #36 | GUU GGA GCU GAU GGC GUA GUC AAG A | UCU UGA CUA CGC CAU CAG CUC CAA C | 25 | - | - | - | - |
| 24 | Exollence_25nt #37 | GUU GGA GCU GAU GGC GUA GUC AAG A | UCU UGA CUA CGC CAU CAG CUC CAA C | 25 | 3 | - | - | - |
| 25 | Exollence_25nt #38 | GUU GGA GCU GAU GGC GUA GUC AAG A | UCU UGA CUA CGC CAU CAG CUC CAA C | 25 | 4 | - | - | - |
| 26 | Exollence_25nt #40 | AGU UGG AGC UGA UGG CGU A | UAC GCC AUC AGC UCC AAC U=tt | 19 | - | - | tt | - |
| 27 | Exollence_25nt #41 | AGU UGG AGC UGA UGG CGU A | UAC GCC AUC AGC UCC AAC U=t*t | 19 | - | - | tt | + |
| 28 | Exollence_25nt #42 | AGU UGG AGC UGA UGG CGU AGG CAA G | CUU GCC UAC GCC AUC AGC UCC AAC U=tt | 25 | - | - | tt | - |
| 29 | Exollence_25nt #44 | AGU UGG AGC UGA UGG CGU AGG CAA G | CUU GCC UAC GCC AUC AGC UCC AAC U=tt | 25 | 3 | - | tt | - |

FIG. 1E

| | ID | SS sequence | AS sequence |
|---|---|---|---|
| 1 | unmodified siKRAS G12D | GUU GGA GCU GAU GGC GUA G=tt | CUA CGC CAU CAG CUC CAA C=tt |
| 2 | unmodified siKRAS G12D-2 | AGU UGG AGC UGA UGG CGU A=tt | UAC GCC AUC AGC UCC AAC U=tt |
| 3 | unmodified siKRAS G12D-PS | GUU GGA GCU GAU GGC GUA G=t*t | CUA CGC CAU CAG CUC CAA C=t*t |
| 4 | unmodified siKRAS G12D-PS-2 | AGU UGG AGC UGA UGG CGU A=t*t | UAC GCC AUC AGC UCC AAC U=t*t |
| 5 | Pattern_unmodified siKRAS G12D | GUU GGA GCU GAU GGC GUA GGC AAG A | UCU UGC CUA CGC CAU CAG CUC CAA C=tt |
| 6 | Pattern_OME modified siKRAS G12D | GUU GGA GCU GAU GGC GUA GGC AAG A | UCU UGC CUA CGC CAU CAG CUC CAA C=tt |
| 7 | Exollence_25nt #3 | GUU GGA GCU GAU GGC GUA GGC AAG A | UCU UGC CUA CGC CAU CAG CUC CAA C=t*t |
| 8 | Exollence_25nt #4 | GUU GGA GCU GAU GGC GUA GGC AAG A=tt | UCU UGC CUA CGC CAU CAG CUC CAA C=tt |
| 9 | Exollence_25nt #5 | GUU GGA GCU GAU GGC GUA G | CUA CGC CAU CAG CUC CAA C=tt |
| 10 | Exollence_25nt #6 | GUU GGA GCU GAU GGC GUA GGC AAG A | UCU UGC CUA CGC CAU CAG CUC CAA C=t*t |
| 11 | Exollence_25nt #7 | GUU GGA GCU GAU GGC GUA GAC AAG A | UCU UGU CUA CGC CAU CAG CUC CAA C=tt |
| 12 | Exollence_25nt #8 | GUU GGA GCU GAU GGC GUA GGC AAG A | UCU UGC CUA CGC CAU CAG CUC CAA C=tt |
| 13 | Exollence_25nt #9 | GUU GGA GCU GAU GGC GUA GGC AAG A | UCU UGC CUA CGC CAU CAG CUC CAA C=t*t |
| 14 | Exollence_25nt-#12 | GUU GGA GCU GAU GGC GUA GAC AAG A | UCU UGU CUA CGC CAU CAG CUC CAA C=tt |
| 15 | Exollence_25nt #13 | GUU GGA GCU GAU GGC GUA GAC AAG A | UCU UGU CUA CGC CAU CAG CUC CAA C=t*t |
| 16 | Exollence_25nt #16 | GUU GGA GCU GAU GGC GUA GAC AAG A | UCU UGU CUA CGC CAU CAG CUC CAA C=tt |
| 17 | Exollence_25nt #28 | GUU GGA GCU GAU GGC GUA GGC AAG A | UCU UGC CUA CGC CAU CAG CUC CAA C |
| 18 | Exollence_25nt #29 | GUU GGA GCU GAU GGC GUA GGC AAG A | UCU UGC CUA CGC CAU CAG CUC CAA C |
| 19 | Exollence_25nt #30 | GUU GGA GCU GAU GGC GUA GGC AAG A | UCU UGC CUA CGC CAU CAG CUC CAA C |
| 20 | Exollence_25nt #32 | GUU GGA GCU GAU GGC GUA GAC AAG A | UCU UGU CUA CGC CAU CAG CUC CAA C |
| 21 | Exollence_25nt #33 | GUU GGA GCU GAU GGC GUA GAC AAG A | UCU UGU CUA CGC CAU CAG CUC CAA C |
| 22 | Exollence_25nt #34 | GUU GGA GCU GAU GGC GUA GAC AAG A | UCU UGU CUA CGC CAU CAG CUC CAA C |
| 23 | Exollence_25nt #36 | GUU GGA GCU GAU GGC GUA GUC AAG A | UCU UGA CUA CGC CAU CAG CUC CAA C |
| 24 | Exollence_25nt #37 | GUU GGA GCU GAU GGC GUA GUC AAG A | UCU UGA CUA CGC CAU CAG CUC CAA C |
| 25 | Exollence_25nt #38 | GUU GGA GCU GAU GGC GUA GUC AAG A | UCU UGA CUA CGC CAU CAG CUC CAA C |
| 26 | Exollence_25nt #40 | AGU UGG AGC UGA UGG CGU A | UAC GCC AUC AGC UCC AAC U=tt |
| 27 | Exollence_25nt #41 | AGU UGG AGC UGA UGG CGU A | UAC GCC AUC AGC UCC AAC U=t*t |
| 28 | Exollence_25nt #42 | AGU UGG AGC UGA UGG CGU AGG CAA G | CUU GCC UAC GCC AUC AGC UCC AAC U=tt |
| 29 | Exollence_25nt #44 | AGU UGG AGC UGA UGG CGU AGG CAA G | CUU GCC UAC GCC AUC AGC UCC AAC U=tt |

EP 4 737 570 A1

**FIG. 2A**

AsPC-1

**FIG. 2B**

HPAFII

FIG. 2C

PANC-1

**FIG. 2D**

**FIG. 2E**

AsPC-1

FIG. 2F

HPAFII

**FIG. 2G**

PANC-1

FIG. 3A

SNU-C2B

**FIG. 3B**

LS174T

**FIG. 3C**

LS513

**FIG. 3D**

**FIG. 3E**

SNU-C2B

**FIG. 3F**

LS174T

**FIG. 3G**

LS513

**FIG. 4A**

**FIG. 4B**

**FIG. 5A**

**FIG. 5B**

## INTERNATIONAL SEARCH REPORT

| International application No. |
| --- |
| **PCT/KR2024/009133** |

| | |
| --- | --- |
| **A.** | **CLASSIFICATION OF SUBJECT MATTER** |
| | **C12N 15/113**(2010.01)i |

According to International Patent Classification (IPC) or to both national classification and IPC

| | |
| --- | --- |
| **B.** | **FIELDS SEARCHED** |

Minimum documentation searched (classification system followed by classification symbols)

C12N 15/113(2010.01); A61K 45/06(2006.01); A61K 47/30(2006.01); A61K 47/54(2017.01); A61K 9/127(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: siRNA, 변형(modification), KRAS G12D, 패턴(pattern), tt 오버행(tt overhang), 포스포로티오에이트(phosphorothioate), 2'-O-메틸화(2'-O-methylation), 암(cancer)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | US 2019-0117570 A1 (BOARD OF REGENTS, THE UNIVERSITY OF TEXAS SYSTEM) 25 April 2019 (2019-04-25)<br>See claims 1 and 17; paragraphs [0019]-[0020], [0023], [0097], [0165], [0173] and [0184]-[0185]; and SEQ ID NO: 1. | 1,5-9,11-12 |
| A | | 2-4 |
| X | KR 10-2021-0002556 A (BOARD OF REGENTS, THE UNIVERSITY OF TEXAS SYSTEM) 08 January 2021 (2021-01-08)<br>See claims 1, 9, 19-22 and 31-33; paragraphs [0072], [0158]-[0159] and [0164]; and SEQ ID NO: 2. | 1,5-9,11-12 |
| X | US 2015-0050341 A1 (SILENSEED LTD.) 19 February 2015 (2015-02-19)<br>See claim 1; paragraphs [0025], [0091]-[0094], [0097]-[0101], [0106] and [0128]; and SEQ ID NOs: 18, 24 and 41. | 1,5-9,11-12 |

☑ Further documents are listed in the continuation of Box C.    ☑ See patent family annex.

| | |
| --- | --- |
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **07 October 2024** | **07 October 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2024/009133** |

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2018-0052083 A (SAMYANG BIOPHARMACEUTICALS CORPORATION) 17 May 2018 (2018-05-17)<br>    See claims 1 and 7-8; paragraphs [0115] and [0199]-[0215]; and SEQ ID NO: 3. | 1,5-9,11-12 |
| A | OZCAN, G. et al. Preclinical and clinical development of siRNA-based therapeutics. Advanced Drug Delivery Reviews. (electronic publication) 07 February 2015, vol. 87, pp. 108-119.<br>    See entire document. | 1-9,11-12 |
| A | KR 10-2018-0086260 A (NITTO DENKO CORPORATION) 30 July 2018 (2018-07-30)<br>    See entire document. | 1-9,11-12 |

Form PCT/ISA/210 (second sheet) (July 2022)

**INTERNATIONAL SEARCH REPORT**

International application No.

**PCT/KR2024/009133**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT

| International application No. |
| --- |
| **PCT/KR2024/009133** |

**Box No. II       Observations where certain claims were found unsearchable (Continuation of item 2 of first sheet)**

This international search report has not been established in respect of certain claims under Article 17(2)(a) for the following reasons:

1. ☑ Claims Nos.: **10**
   because they relate to subject matter not required to be searched by this Authority, namely:

   Claim 10 pertains to a method for treatment of the human body by therapy, and thus pertains to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/KR2024/009133** |

| Patent document cited in search report | | | Publication date (day/month/year) | Patent family member(s) | | | Publication date (day/month/year) |
| --- | --- | --- | --- | --- | --- | --- | --- |
| US | 2019-0117570 | A1 | 25 April 2019 | AU | 2016-275046 | A1 | 04 January 2018 |
| | | | | AU | 2016-275046 | B2 | 28 July 2022 |
| | | | | BR | 112017026467 | A2 | 11 September 2018 |
| | | | | CA | 2988585 | A1 | 15 December 2016 |
| | | | | CN | 107980004 | A | 01 May 2018 |
| | | | | CN | 113694075 | A | 26 November 2021 |
| | | | | EP | 3307890 | A1 | 18 April 2018 |
| | | | | IL | 256175 | A | 28 February 2018 |
| | | | | IL | 256175 | B1 | 01 June 2024 |
| | | | | JP | 2018-520125 | A | 26 July 2018 |
| | | | | JP | 2021-107463 | A | 29 July 2021 |
| | | | | JP | 2024-023853 | A | 21 February 2024 |
| | | | | KR | 10-2018-0017119 | A | 20 February 2018 |
| | | | | US | 10959952 | B2 | 30 March 2021 |
| | | | | US | 2018-0177727 | A1 | 28 June 2018 |
| | | | | WO | 2016-201323 | A1 | 15 December 2016 |
| KR | 10-2021-0002556 | A | 08 January 2021 | AU | 2019-255765 | A1 | 10 December 2020 |
| | | | | CA | 3097558 | A1 | 24 October 2019 |
| | | | | CN | 112512507 | A | 16 March 2021 |
| | | | | EP | 3781131 | A1 | 24 February 2021 |
| | | | | JP | 2021-522164 | A | 30 August 2021 |
| | | | | US | 2021-0115449 | A1 | 22 April 2021 |
| | | | | WO | 2019-204624 | A1 | 24 October 2019 |
| US | 2015-0050341 | A1 | 19 February 2015 | US | 2013-0280329 | A1 | 24 October 2013 |
| | | | | US | 2014-0314854 | A1 | 23 October 2014 |
| | | | | US | 8889642 | B2 | 18 November 2014 |
| | | | | US | 9080173 | B2 | 14 July 2015 |
| | | | | WO | 2013-157010 | A1 | 24 October 2013 |
| KR | 10-2018-0052083 | A | 17 May 2018 | KR | 10-1949507 | B1 | 18 February 2019 |
| | | | | WO | 2018-088719 | A1 | 17 May 2018 |
| KR | 10-2018-0086260 | A | 30 July 2018 | AU | 2016-371624 | A1 | 12 July 2018 |
| | | | | AU | 2016-371624 | B2 | 27 August 2020 |
| | | | | BR | 112018008344 | A2 | 30 October 2018 |
| | | | | CA | 3005937 | A1 | 22 June 2017 |
| | | | | CA | 3005937 | C | 09 November 2021 |
| | | | | CN | 108367022 | A | 03 August 2018 |
| | | | | EP | 3386519 | A1 | 17 October 2018 |
| | | | | EP | 3386519 | B1 | 24 March 2021 |
| | | | | EP | 3816287 | A1 | 05 May 2021 |
| | | | | JP | 2018-537104 | A | 20 December 2018 |
| | | | | JP | 6457704 | B2 | 23 January 2019 |
| | | | | US | 10358647 | B2 | 23 July 2019 |
| | | | | US | 11359202 | B2 | 14 June 2022 |
| | | | | US | 11390871 | B2 | 19 July 2022 |
| | | | | US | 11926831 | B2 | 12 March 2024 |
| | | | | US | 2017-0166898 | A1 | 15 June 2017 |
| | | | | US | 2019-0323015 | A1 | 24 October 2019 |
| | | | | US | 2022-0267779 | A1 | 25 August 2022 |
| | | | | WO | 2017-106111 | A1 | 22 June 2017 |

Form PCT/ISA/210 (patent family annex) (July 2022)

**EP 4 737 570 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230083349
- KR 1020230083355
- KR 1020230083360
- KR 1020240084878

**Non-patent literature cited in the description**

- Remington's Pharmaceutical Science. Mack Publishing Company